# EUROPEAN PATENT SPECIFICATION

(11) **EP 4 351 506 B1**
(45) Date of publication and mention of the grant of the patent: **01.04.2026**
(21) Application number: 22821132.2
(22) Date of filing: 10.06.2022
(51) Int. Cl.: A61K 8/00, A61K 8/02, A61K 8/30, A61K 8/18, A61P 17/00, A61K 31/11, A61K 31/12, A61K 47/14, A61K 8/04, A61K 8/34, A61K 8/37, A61K 8/49, A61K 9/00, A61K 9/14, A61Q 19/00, A61K 8/35

(54) **LIPID CRYSTALLINE COMPOSITIONS WITH ENHANCED STABILITY FOR TOPICAL DELIVERY OF ACTIVE AGENT COMBINATIONS**
LIPID-KRISTALLINE ZUSAMMENSETZUNGEN MIT VERBESSERTER STABILITÄT ZUR TOPISCHEN VERABREICHUNG VON WIRKSTOFFKOMBINATIONEN
COMPOSITIONS CRISTALLINES LIPIDIQUES À STABILITÉ AMÉLIORÉE POUR ADMINISTRATION TOPIQUE DE COMBINAISONS D'AGENTS ACTIFS

(30) Priority: 11.06.2021 US 202163209676 P
(43) Date of publication of application: 17.04.2024
(73) Proprietor: Ankh Life Sciences Limited, Dublin 2, D02 DK18 (IE)
(72) Inventor: ZAID, Gene H., Hutchinson, Kansas 67502 (US); LINDAL, Åke Richard, 217 75 Malmö (SE); NILSSON, Anette Susanne, 237 34 Bjärred (SE); WEST, Cameron E., Hutchinson, Kansas 67502 (US); PRONIUK, Stefan, Austin, Texas 78746 (US); MOORE, Robert Preston, Great Bend, Kansas 67530 (US)
(74) Representative: Uexküll & Stolberg
(86) International application number: PCT/US2022/033056
(87) International publication number: WO 2022/261466

(56) References cited:
- US-A- 6 156 294
- US-A1- 2016 081 968
- US-A1- 2016 089 333
- US-A1- 2017 042 865
- US-A1- 2018 338 916

## Description

### CROSS-REFERENCE TO RELATED APPLICATIONS

The present application claims the priority benefit of U.S. Provisional Patent Application Serial No. 63/209,676, filed June 11, 2021, entitled TOPICAL FORMULATIONS FOR DELIVERY OF THERAPEUTIC AGENTS.

### BACKGROUND OF THE INVENTION

### Field of the Invention

The present invention relates to compositions with enhanced stability of active agents while maintaining therapeutic activity of the agents for topical delivery.

### Description of Related Art

The use of herbal remedies and the subsequent analysis of active ingredients in them has been important in the formation of many modern drugs, including the vinca alkaloids, paclitaxel and etoposide. However, stability of active ingredients derived from plants and herbs remains a continuing challenge. It is a particular challenge to formulate combinations of active ingredients into topical compositions (e.g., ointments, creams, etc.) which require some term of storage stability, without degradation of such ingredients. In contrast, formulations with storage stability may be achieved, but at the cost of decreased therapeutic efficacy and/or skin penetration efficacy.

### SUMMARY OF THE INVENTION

The present invention is broadly concerned with stabilized topical formulations, specifically a water-based cream comprising lipid crystals and the active agents dispersed therein. As used herein, references to "topical" encompass formulations intended to be applied for cutaneous or dermatological skin conditions, epithelial related conditions, and/or for cosmetic purposes, as well as semimucous membranes (e.g., lips) and/or mucous membranes. Thus, use of the formulations and methods of treatment involve locally applying (e.g., by spreading or spraying) the formulations to the surface of a body part (e.g., a skin surface) to be treated in a desired thickness allowing the formation of a film, and leaving the formulations in contact with the part for a sufficient period to obtain the desired cosmetic or dermatological effect (e.g., for penetration of the active agents through the skin). It is contrasted with systemic administration of active ingredients, such as by injection, intravenous infusion, oral administration, and the like.

The active agents comprise one or more curcumin component(s), harmine component(s), and isovanillin component(s), and sub-combinations thereof. Preferably, the active agents comprise respective quantities of at least two of, curcumin component(s), harmine component(s), and isovanillin component(s). The preferred topical formulations include all three of these components, but sub-combinations thereof are also useful, i.e., topical formulations comprising curcumin component(s) and harmine component(s), curcumin component(s) and isovanillin component(s), and harmine component(s) and isovanillin component(s). In particularly preferred embodiments, the active components of the compositions (i.e., those having a significant therapeutic effect) consist essentially of curcumin, harmine, and isovanillin components in the case of three-component compositions, and consist essentially of two of the three components in the case of the two-component compositions. Preferably, the at least one curcumin component comprises curcumin, the at least one harmine component comprises harmine, and the at least one isovanillin component comprises isovanillin or vanillin.

Several attempts have been made to stabilize formulations containing one or more of the three agents without success. Curcuminoids, are, in particular difficult to stabilize. Many unsuccessful attempts have also been made to facilitate skin penetration of the drugs. In general, most compounds are more stable or easier to stabilize in the solid state while skin penetration requires the active compounds in solution. Thus, topical formulations wherein these active agents are both stabilized, but remain available for skin penetration has not previously been achieved.

In the inventive crystalline lipid formulation all three active agents are combined to a chemically and physically stable formulation. This has been achieved by using a cream formulation that is solid, crystalline at ambient temperature and a fluid crystalline, at skin temperature. This formulation comprises at least one monoglyceride of a fatty acid in crystalline form, preferably C12 and C₁₄ monoglycerides, more preferably GRAS as distilled monoglycerides of sunflower oil, that is transformed to solid crystals during manufacture. Thus, the formulation comprises a suspension of solid lipid crystals in an aqueous solution that crystallizes and melts at skin temperature. The formulation can stabilize sensitive compounds and enable encapsulation of hydrophobic active compounds in the lipids. When a chemical substance is in solid form the chemical stability is high since reactions causing poor stability of the active require an active agent in dissolved state, otherwise no reactions can occur. Another aspect is the transport, diffusion, of reactants and other participants in the degradation reactions. When the diffusion of these compounds can be kept at a minimum, the degradation rate will be kept low.

The crystalline lipid formulation has been demonstrated as being both stable and delivering actives in *in vitro* and *in vivo* model experiments. Methods of manufacture are also described herein. In general, the methods comprise forming an aqueous solution with a buffer, which is heated to at least 70°C, followed by the addition of at least one monoglyceride of a fatty acid in crystalline form to form a mixture. The mixture of monoglycerides and water form multilamellar vesicles at about 70°C. The temperature is suitable for melting the monoglyceride. The active agents are then added and the composition is allowed to cool under ambient conditions to provide for a controlled rate of cooling to form lipid crystals. At 50°C and below, fluid lamellar crystals are formed. In some embodiments, the active agents can be added after the composition is allowed to partially cool, but before crystals are formed (e.g., about 35-45°C). For most applications, it has been found that the solution only needs be heated to about 70°C to melt the crystalline monoglycerides. At 30 to 35°C the fluid crystals solidify containing a lipid layer of about 60 Å and a water layer of 20 Å. This large negatively charged surface can slow redox reactions by adhering intermediaries in the reaction. Lipid surfaces presented to the monoglycerides during manufacture are then covered with bilayers of mono-glycerides excluding water from the surface and encapsulating the active agent.

The cooling can be conducted as desired to provide the desired size crystals. Preferably, the cooling is conducted at a fixed rate of about 0.5 to 5° C per minute, until crystallization begins, which is usually about 30 to 39° C. When crystallization is completed to a desired level, the dispersion can be further cooled to room temperature (~25+/-2°C). The resulting composition exhibits significantly enhanced stabilization of the active agents compared to common emulsions or dispersions. Furthermore, the composition is capable of maintaining a therapeutically-effective concentration of active agents for at least 6 months of storage at ambient conditions, preferably after at least 24 months of storage in a sealed container at ambient conditions (standard room temperature/humidity, RTH, e.g., ~25+/-2°C, 40-65% relative humidity). Thus, the composition is capable of meeting or exceeding requirements for use as a topical pharmaceutical agent comprising the active agents.

### BRIEF DESCRIPTION OF THE DRAWINGS

Figure (Fig.) 1 is a graph showing the single API Efficacy Compared to GZ17-6.02 Combination.
Fig. 2 is a graph showing Isovanillin and Isovanillin Doublets Efficacy Compared to GZ17-6.02 Combination.
Fig. 3 is a graph showing Harmine and Harmine Doublets Efficacy Compared to GZ17-6.02 Combination.
Fig. 4 is a graph showing Curcumin and Curcumin Doublets Efficacy Compared to GZ17-6.02 Combination.
Fig. 5 is a graph showing dose Response of API and GZ17-6.02.
Fig. 6 is a graph showing In vitro Efficacy comparison of GZ17-6.02 and 5FU.
Fig. 7 shows photographs of the gel, emulsion, and cream formulations.
Fig. 8 shows SEM Images at 100 um of the monoglyceride cream.
Fig. 9 is a graph of the stability assay showing the detectable levels of (A) Curcumin, (B) Isovanillin, and (C) Harmine, over time in different formulations.
Fig. 10 is a graph of the stability assay showing the degradation products of (A) Curcumin, (B) Isovanillin, and (C) Harmine over time in different formulations.
Fig. 11 is a graph showing the cumulative amount (µg/cm2) of Isovanillin permeated though pig skin from the three investigated formulations. Mean of 4-5 replicates are given, SD are used as errors.
Fig. 12 is a graph showing the flux of Isovanillin (µg/cm2/h) though pig skin membranes from the three investigated formulations. Mean of 4-5 replicates are given, SD are used as errors.
Fig. 13 is a graph showing the cumulative percentage (%) of applied Isovanillin permeated though pig skin membranes from the three investigated formulations. Mean of 4-5 replicates are given, SD are used as errors.
Fig. 14 is a graph showing the amount of Isovanillin (µg/g) accumulated in the skin membranes. Mean of 3-5 replicates are given, SD are used as errors.
Fig. 15 is a graph showing the cumulative amount (µg/cm2) of Harmine permeated though pig skin from the three investigated formulations. Mean of 4-5 replicates are given, SD are used as errors.
Fig. 16 is a graph showing the flux of Harmine (µg/cm2/h) though pig skin membranes from the three investigated formulations. Mean of 4-5 replicates are given, SD are used as errors.
Fig. 17 is a graph showing the cumulative percentage (%) of applied Harmine permeated though pig skin membranes from the three investigated formulations. Mean of 4-5 replicates are given, SD are used as errors.
Fig. 18 is a graph showing the amount of Harmine (µg/g) accumulated in the skin membranes. Mean of 4-5 replicates are given, SD are used as errors.
Fig. 19 is a graph showing the amount of Harmine accumulated in the skin membranes. Mean of 3-5 replicates are given, SD are used as errors.
Fig. 20 is a graph showing the amount of Curcumin (µg/g) accumulated in the skin membranes. Mean of 3-5 replicates are given, SD are used as errors.
Fig. 21 is a graph showing the cumulative amount (µg/cm2) of Isovanillin permeated after application of 6% GZ17-6.02 monoglyceride cream and 3% GZ17-6.02 monoglyceride cream. Standard deviation is given as error bars.
Fig. 22 is a graph showing the cumulative percentage (%) of applied dose of Isovanillin permeated after application of 6% GZ17-6.02 monoglyceride cream and 3% GZ17-6.02 monoglyceride cream. Standard deviation is given as error bars.
Fig. 23 is a graph showing the amount of Isovanillin in epidermis after application of 3% GZ17-6.02 monoglyceride cream and 6% GZ17-6.02 monoglyceride cream. Mean value with standard deviation as error bars are displayed.
Fig. 24 is a graph showing the amount of Harmine in epidermis after application of 3% GZ17-6.02 monoglyceride cream and 6% GZ17-6.02 monoglyceride cream. Mean value with standard deviation as error bars are displayed.
Fig. 25 is a graph showing the amount of Curcumin in epidermis after application of 3% GZ17-6.02 monoglyceride cream and 6% GZ17-6.02 monoglyceride cream. Mean value with standard deviation as error bars are displayed.
Fig. 26 is a graph showing the amount of Isovanillin in dermis after application of 3% GZ17-6.02 monoglyceride cream and 6% GZ17-6.02 monoglyceride cream. Mean value with standard deviation as error bars are displayed.
Fig. 27 is a graph showing the amount of Harmine in dermis after application of 3% GZ17-6.02 monoglyceride cream and 6% GZ17-6.02 monoglyceride cream. Mean value with standard deviation as error bars are displayed.
Fig. 28 is a graph showing the amount of Curcumin in dermis after application of 3% GZ17-6.02 monoglyceride cream (blue bar) and 6% GZ17-6.02 monoglyceride cream (red bar). Mean value with standard deviation as error bars are displayed.
Fig. 29 shows graphs from the AK studies showing (A)-(B) cell death of the formulations with individual, combined, and 3-component formulations were tested, and (C) the genotoxicity of each test composition.
Fig. 30 shows three graphs from Gene Set Variation Analysis (GSVA) of the up/down regulation of various pathways by the compounds.
Fig. 31 is a heat map of the inducement or inhibition of certain protein markers via proteomics analysis using the monoglyceride formulation (GZ21T) as compared to a control.

### DETAILED DESCRIPTION

In more detail, described herein are novel formulations that demonstrate the ability to stabilize two or more active compounds, with limited water solubility, which can be simultaneously encapsulated in the invented formulation technology. This means that unstable and otherwise previously "incompatible" compounds can be included in the same formulation. Without limiting the scope of the invention, one particularly useful active agent combination is the three compounds isovanillin, harmine and curcumin.

*Arum (A.) palaestinum*, a member of the Araceae family of plants, is a foundation of traditional medicine in treating disorders ranging from stomach upset to cancer. *A. palaestinum* extracts containing isovanillin, were combined with other plant extracts and studied. Additional work identified a three-component mixture which showed clear preclinical synergy between the three components, isovanillin identified in *A. palaestinum*, harmine identified in *Peganum harmala* and curcumin identified in *Curcuma longa.* Further pre-clinical evaluation of these components demonstrated if any one of the three components was removed, the anticancer effects were diminished.

A synthetic mixture of the three components, named GZ17-6.02, has been prepared for further development as an orally administered treatment for patients with histologically confirmed advanced solid tumors or lymphomas. The proportions of the active components isovanillin, harmine, and curcumin in the GZ17-6.02 drug substance are 77%, 13% and 10% by weight, respectively. In preclinical studies, GZ17-6.02 has demonstrated a synergistic anti-proliferative activity against a variety of human solid tumor, multiple myeloma and lymphoma cell lines in vitro, and efficacy in human pancreatic cancer, head and neck squamous cell carcinoma (HNSCC) and colorectal adenocarcinoma using orthotopic or subcutaneous athymic mouse models.

Further preclinical investigation and evidence has demonstrated GZ17-6.02's potential to treat dermatologic disease if applied as a topical formulation. Initial preclinical research led to the exploration of a topical formulation to treat actinic keratosis (AK), basal cell carcinoma, and various forms of cutaneous t-cell lymphoma (CTCL), including mycosis fungoides. However, formulation of GZ17-6.02 as a topical remained elusive, as the stability and therapeutic activity of these powerful ingredients was significantly impacted during storage. The novel formulation strategies have been used to develop a topical lipid crystalline cream, which has been exemplified using the same stoichiometric ratio by weight of isovanillin, harmine and curcumin solubilized in a novel cream formulation. This treatment approach is supported by preclinical data demonstrating effect in various signaling pathways often mutated in these dermatological diseases.

### The Curcumin-Harmine-Isovanillin Compositions

U.S. Patent No. 10,092,550 relating to the GZ17-6.02 drug describes a variety of components containing curcumin, harmine, and isovanillin, and uses thereof. These compositions generally include respective quantities of curcumin, harmine, and isovanillin, and are typically, though not necessarily, in powdered form for formulation with the lipid crystalline base.

WO 2017/042865 describes therapeutic compositions comprising at least two, and preferably all three, of the following components: curcumin, harmine, and isovanillin. These compositions demonstrate synergistic anti-cancer activity across a wide range of human cancers. The formulations are suitable for various delivery methods and offer improved therapeutic potential by targeting both cancer cells and cancer stem cells.

Curcumin (diferuloylmethane, 1,7-bis(4-hydroxy3-methoxyphenyl)-1,6-heptadiene-3,5-dione) is a symmetrical diaryl heptanoid. It occurs as a part of a curcuminoid plant extract containing curcumin, demethoxycurcumin, and bis-demethoxycurcumin.

| | |
|---|---|
| CAS Number: 458-37-7 | 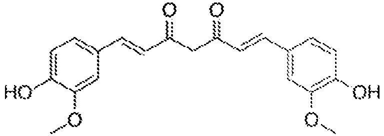 |
| Molecular Formula: | C21H206 |
| Molecular Weight: | 368.39 |

It exists in solution as an equilibrium mixture of the symmetrical dienone (diketo) and the keto-enol tautomer; the keto-enol form is strongly favored by intramolecular hydrogen bonding. 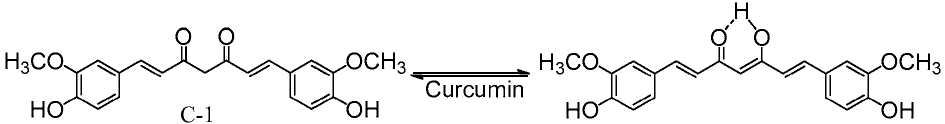 Curcumin contains two aryl rings separated by an unsaturated 7-carbon linker having a symmetrical β-diketone group (as used herein, "β-diketone" embraces both tautomeric forms, namely the diketo and enol forms). The aryl rings of curcumin contain a hydroxyl group in the para position and a methoxy group in the *meta* position.

Harmine (7-methoxy-1-methyl-9H-pyrido[3,4-b]indole, aka 1-methyl-7-methoxy-β-carboline) is a methoxy methyl pyrido indole belonging to the β-carboline family of compounds.

| | |
|---|---|
| CAS Number: 441-51-3 | 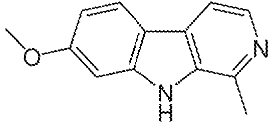 |
| Molecular Formula: | C13H12N2O |
| Molecular Weight: | 212.25 |

Isovanillin (CAS #621-59-0) is a phenolic aldehyde vanillin isomer, and has the molecular formula C8H8O3. The vanillin compound(s) useful in the invention are phenyl aldehydes, and one family of such compounds have the structure 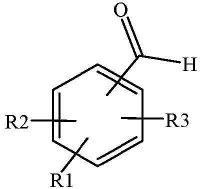 where R1 is selected from the group consisting of OH, H, C1-C4 alkoxy groups, F, Cl, Br, I, N, and NO2, and R2 and R3 are independently selected from the group consisting of H, OH, and C1-C4 alkoxy groups, it being understood that the aldehyde group and R1, R2, and R3 can be located at any position around the phenyl ring.

Certain specific vanillin compounds are vanillin, isovanillin, orthovanillin, and include the following exemplary vanillin compounds:

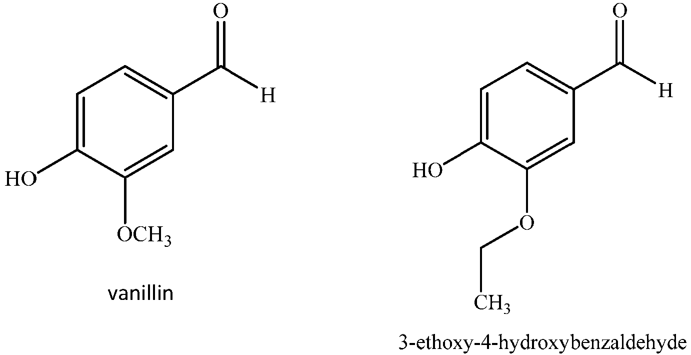

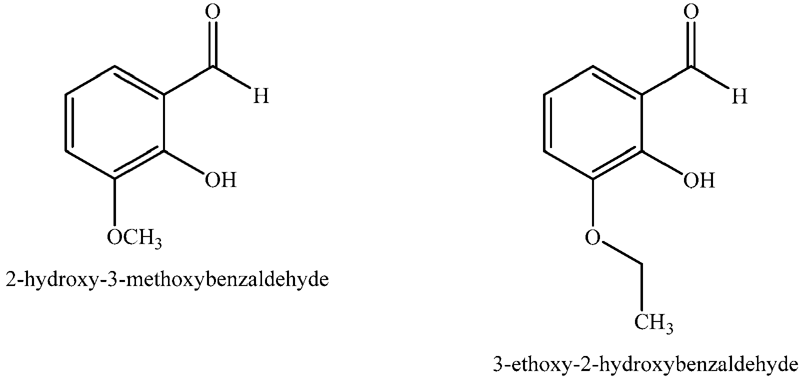

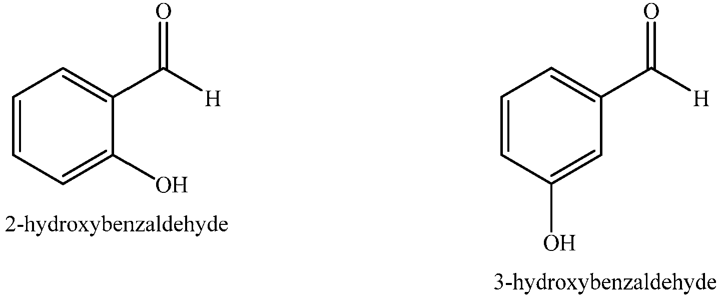

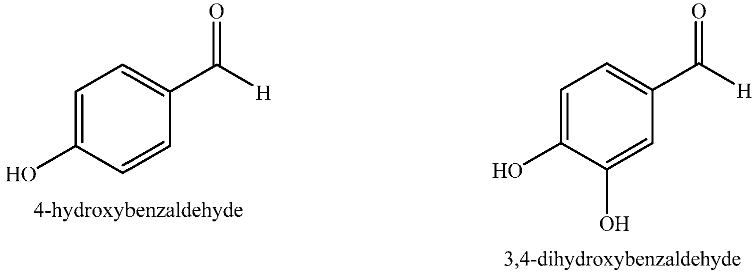

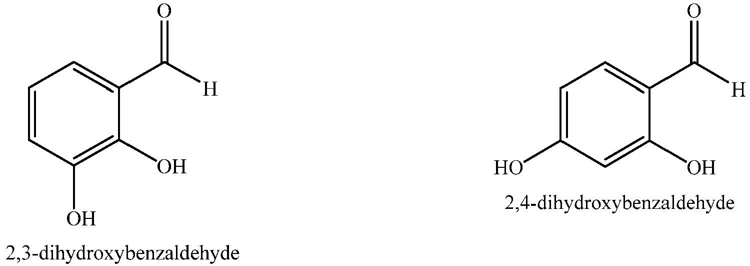

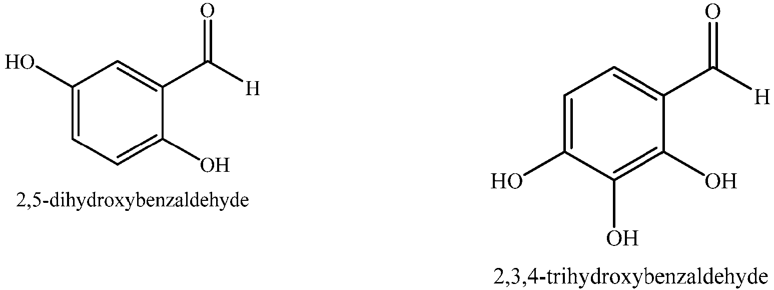

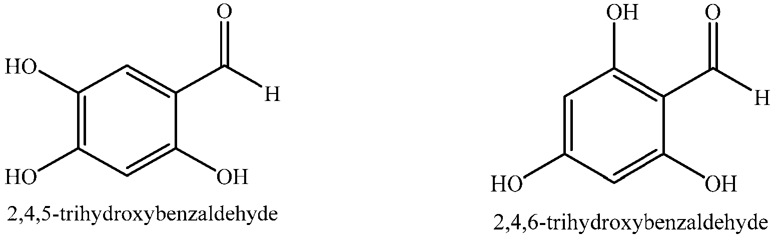

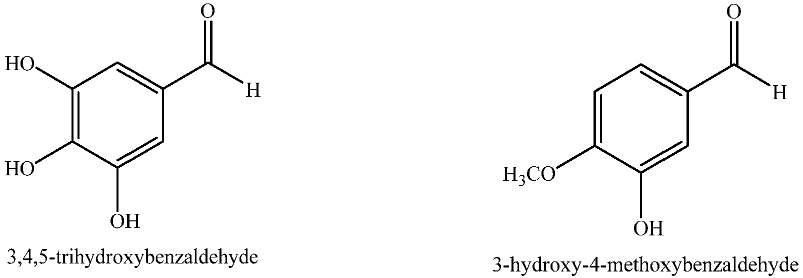

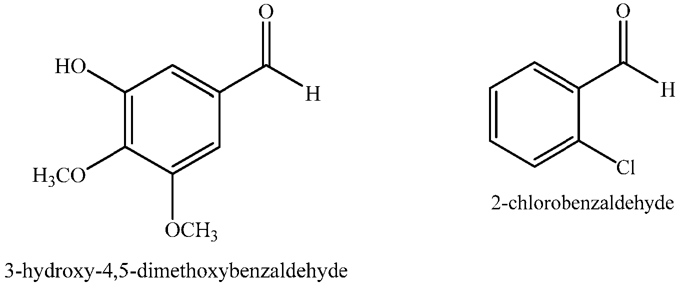

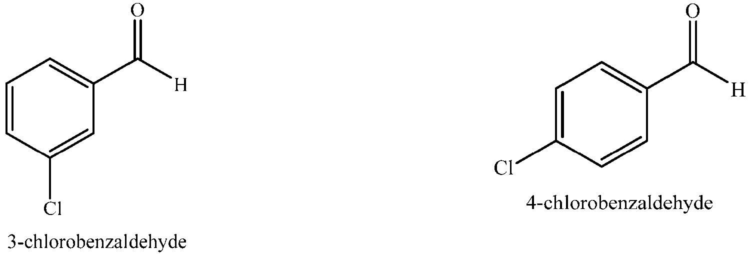

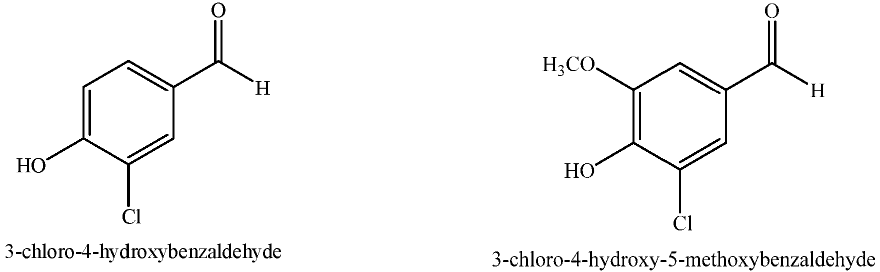

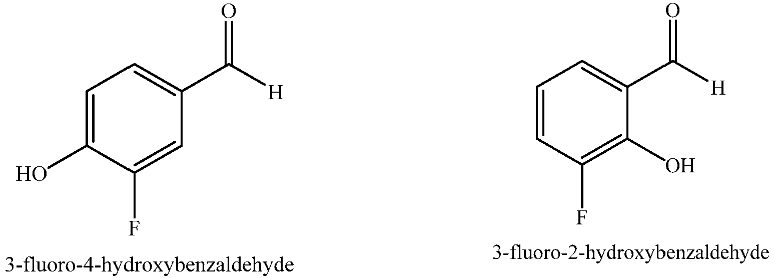

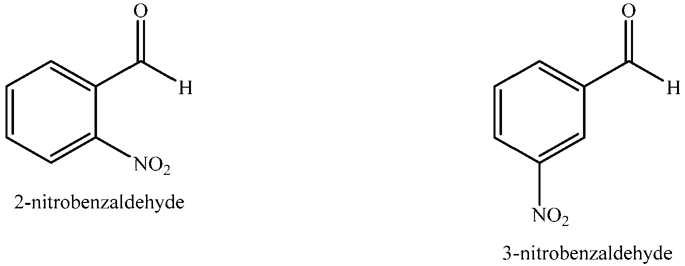

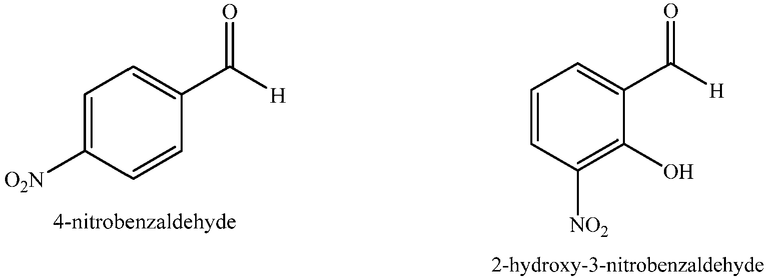

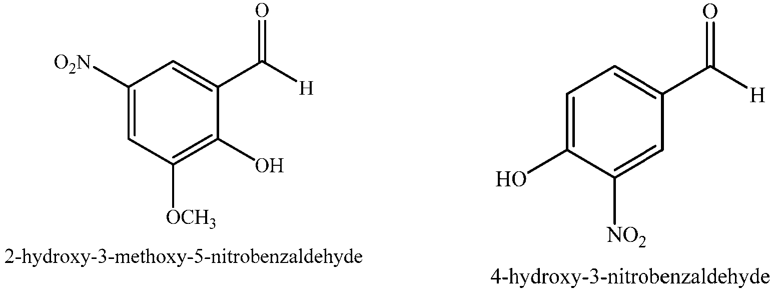

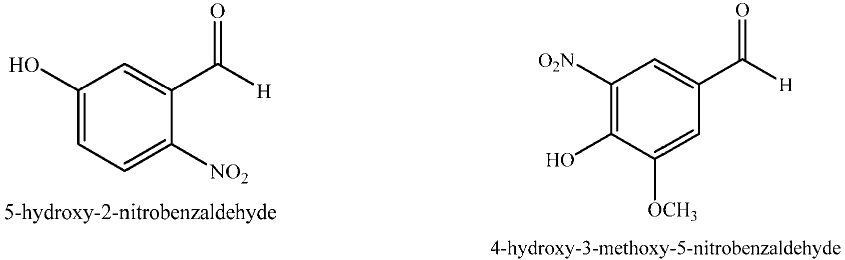

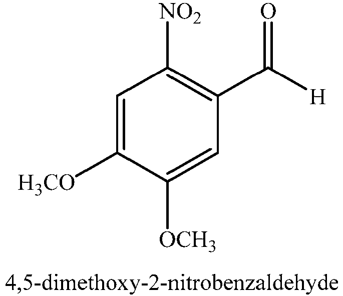

Thus, as used herein, "curcumin," "harmine," and "isovanillin" respectively refer to the above-identified compounds as well as the isomers, tautomers, derivatives, solvates, degradation products, metabolites, esters, metal complexes (e.g., Cu, Fe, Zn, Pt, V), prodrugs, and pharmaceutically acceptable salts thereof. As used herein, a derivative is a compound that can be imagined to arise or actually be synthesized from a parent compound by replacement of one atom with another atom or a group of atoms. Similarly, pharmaceutically acceptable salts with reference to the components of the composition means salts which are pharmaceutically acceptable, e.g., salts which are useful in preparing pharmaceutical compositions that are generally safe, non-toxic, and neither biologically nor otherwise undesirable and are acceptable for human pharmaceutical use, and which possess the desired degree of pharmacological activity. Such pharmaceutically acceptable salts may include acid addition salts formed with organic or inorganic acids, and base addition salts.

In one or more embodiments, the individual components are naturally or synthetically derived, and should have purities of at least about 90% by weight, and most preferably at least about 98% by weight.

In the three-component active agents, isovanillin would normally be the predominant ingredient on a weight basis, with harmine and curcumin being present in lesser amounts on a weight basis. Generally, isovanillin should be present at a level of at least about three times (more preferably at least about five times) greater than that of each of harmine and curcumin, again on a weight basis. The as-added amounts of the components should give weight ratios of from about 0.1-25.0:0.1-5:0.1-5 (isovanillin:harmine:curcumin), and more preferably from about 10:1.7:0.85. In terms of amounts of the three components, isovanillin should be present at a level of from about 25-85% by weight, harmine at a level of from about 7-50% by weight, and curcumin at a level of from about 5-40% by weight, all based upon the total weight of the three ingredients taken as 100% by weight.

"Pharmaceutically acceptable salts" with reference to the components means salts of the components which are pharmaceutically acceptable, i.e., salts which are useful in preparing pharmaceutical compositions that are generally safe, non-toxic, and neither biologically nor otherwise undesirable and are acceptable for human pharmaceutical use, and which possess the desired degree of pharmacological activity. Such pharmaceutically acceptable salts include acid addition salts formed with inorganic acids such as hydrochloric acid, hydrobromic acid, sulfuric acid, nitric acid, phosphoric acid, and the like; or with organic acids such as 1,2-ethanedisulfonic acid, 2-hydroxyethanesulfonic acid, 2-naphthalenesulfonic acid, 3-phenylpropionic acid, 4,4'-methylenebis(3-hydroxy-2-ene-1-carboxylic acid), 4-methylbicyclo[2.2.2]oct-2-ene-1-carboxylic acid, acetic acid, aliphatic mono- and dicarboxylic acids, aliphatic sulfuric acids, aromatic sulfuric acids, benzenesulfonic acid, benzoic acid, camphorsulfonic acid, carbonic acid, cinnamic acid, citric acid, cyclopentanepropionic acid, ethanesulfonic acid, fumaric acid, glucoheptonic acid, gluconic acid, glutamic acid, glycolic acid, heptanoic acid, hexanoic acid, hydroxynaphthoic acid, lactic acid, laurylsulfuric acid, maleic acid, malic acid, malonic acid, mandelic acid, methanesulfonic acid, muconic acid, o-(4-hydroxybenzoyl)benzoic acid, oxalic acid, p-chlorobenzenesulfonic acid, phenyl-substituted alkanoic acids, propionic acid, p-toluenesulfonic acid, pyruvic acid, salicylic acid, stearic acid, succinic acid, tartaric acid, tertiarybutylacetic acid, trimethylacetic acid, and the like. Pharmaceutically acceptable salts also include base addition salts which may be formed when acidic protons present are capable of reacting with inorganic or organic bases. Acceptable inorganic bases include sodium hydroxide, sodium carbonate, potassium hydroxide, aluminum hydroxide and calcium hydroxide. Acceptable organic bases include ethanolamine, diethanolamine, triethanolamine, tromethamine, N-methylglucamine and the like. It should be recognized that the particular anion or cation forming a part of any salt of this invention is not critical, so long as the salt, as a whole, is pharmacologically acceptable. Additional examples of pharmaceutically acceptable salts and their methods of preparation and use are presented in Handbook of Pharmaceutical Salts Properties, and Use, P. H. Stahl & C. G. Wermuth eds., ISBN 978-3-90639-058-1 (2008).

### GZ17-6.02

One three-component active agent combination in accordance with the invention is referred to as "GZ17-6.02," sometimes called "6.02." This combination comprises 77% by weight of 98% pure solid synthetic isovanillin, 13% by weight of 99% pure solid synthetic harmine, and 10% by weight of a commercially available solid curcumin product containing 99.76% by weight curcuminoids, namely 71.38% curcumin, 15.68% demethoxycurcumin, and 12.70% bisdemethoxycurcumin. The solids are thoroughly mixed together to complete the preparation. Thus, the three-component active agent combination consists of individual quantities of normally highly purified curcumin, harmine, and isovanillin components at ratios of approximately 77:13:10 (isovanillin:harmine:curcumin). Each such component may be made up of one or more isovanillin, harmine, and/or curcumin compounds. Generally, it is preferred that the isovanillin component is the preponderant component in the composition on a weight basis, with the harmine and curcumin components being present in lesser amounts on a weight basis. Still further, the isovanillin component may be present at a level of at least three times (more preferably at least five times) greater than that of each of the harmine and curcumin components. In terms of amounts of the three components, the isovanillin component should be present at a level of from about 25-85% by weight, the harmine component should be present at a level of from about 7-50% by weight, and the curcumin component should be present at a level of from about 5-40% by weight, all based on the total weight of the three components taken as 100% by weight. The total active agent content (e.g., of the 2 or 3 combined components) in the composition for a topical formulation can range from about 0.1%-25%, preferably from about 0.5%-20%, more preferably from about 1%-15%, more preferably from about 1.5%-10%, more preferably from about 3%-9% (w/w).

The single most preferred GZ17-6.02 active agent combination, and that tested in the examples, was made by dispersing relative quantities of solid synthetic isovanillin (771 mg, 98% by weight purity), synthetic harmine (130.3 mg, 99% by weight purity), and a commercially available curcumin product derived by the treatment of turmeric (98.7 mg, containing 99.76% by weight curcuminoids, namely 71.38% curcumin, 15.68% demethoxycurcumin, and 12.70% bisdemethoxycurcumin), at a weight ratio of 771:130.3:98.7 (isovanillin:harmine:curcumin product) in the base formulation. In more preferred embodiments, an even more purified curcumin is used that has even higher concentration of curcumin (substantially purified curcumin at 98.7%, preferably ~99% by weight purity, preferably at least 99.9% by weight) and does not contain any appreciable amounts of demethoxycurcumin or bisdemethoxycurcumin.

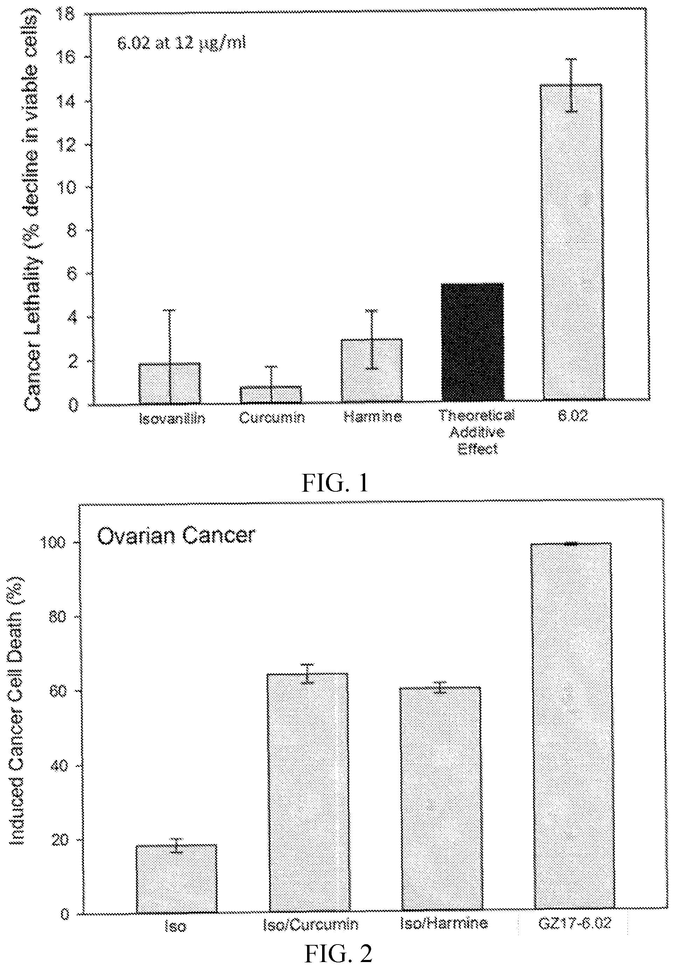

There is an apparent synergy when comparing the efficacy of each individual active pharmaceutical ingredient to the GZ17-6.02 active agent combination. Data comparing isovanillin, harmine, and curcumin individual efficacy to the combination of the three active pharmaceutical ingredients (in ethanol) found within the GZ17-6.02 formulation demonstrated a substantial increase in efficacy. The Fig. below compares the cancer lethality of each active pharmaceutical ingredient found within GZ17-6.02 to the GZ17-6.02 combination itself in an ovarian cancer cell line. GZ17-6.02 was tested at a concentration of 12 ug/ml and each active pharmaceutical ingredient was tested at its corresponding concentration respective of the 12 ug/ml GZ17-6.02 concentration. This results, shown in Fig. 1, illustrate the synergistic effect of the three active pharmaceutical ingredients and the need for inclusion of each active pharmaceutical ingredient.

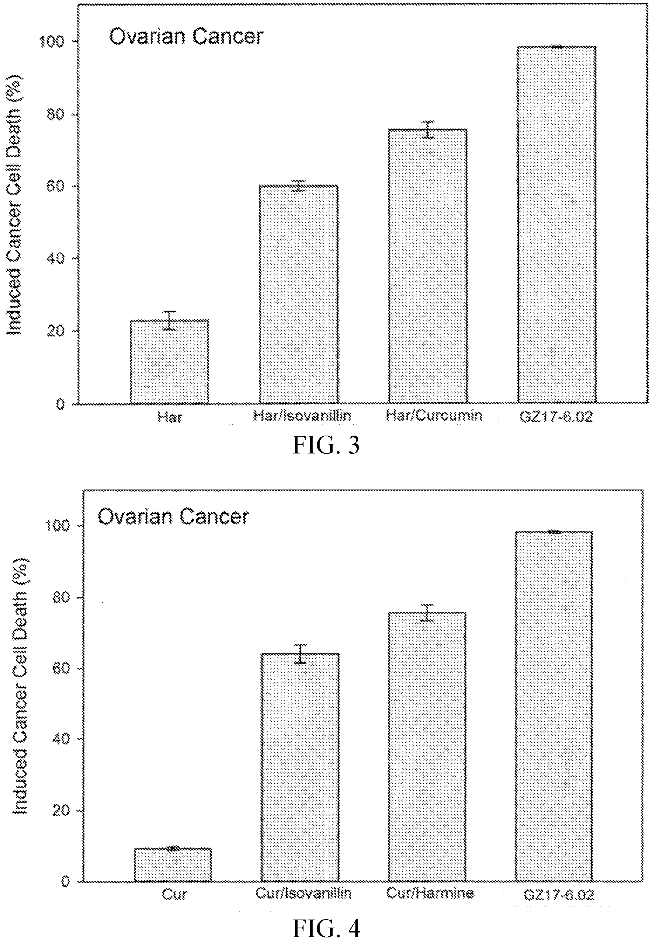

Additional data shown in Figs. 2-4, demonstrates that various doublet combinations of the three active pharmaceutical ingredients, while still effective as compared to individual components, are not as effective as the three active pharmaceutical ingredient combination found in GZ17-6.02. In an ovarian cancer cell line, GZ17-6.02 outperforms various doublet combinations.

This synergistic effect is also demonstrated within the actinic keratosis cell line HaCaT observed by the decreasing amount of each active pharmaceutical ingredient required to achieve IC50 concentrations when comparing individual amounts of isovanllin, harmine, and curcumin to amounts of each active pharmaceutical ingredient found within the GZ17-6.02 formulation. This data supports the premise that multiple active pharmaceutical ingredients are required to attain the maximal desired treatment effect as the IC50 concentrations of each active pharmaceutical ingredient decreased as part of the formulation compared to individual assessment.

**Table: Individual API and GZ17-6.02 IC50 Concentrations**

| Component | IC50 as Monotherapy | IC50 as Combination |
|---|---|---|
| GZ 17-6.02 | N/A | 5.07 ug/ml |
| Isovanillin (MW 152) | Undetermined | 3.904 ug/ml (28.26 uM) |
| Harmine (MW 212) | 2.548 ug/ml (12.02 uM) | 0.659 ug/ml (3.11 uM) |
| Curcumin (MW 368) | 0.736 ug/ml (2.0 uM) | 0.507 ug/ml (1.38 uM) |

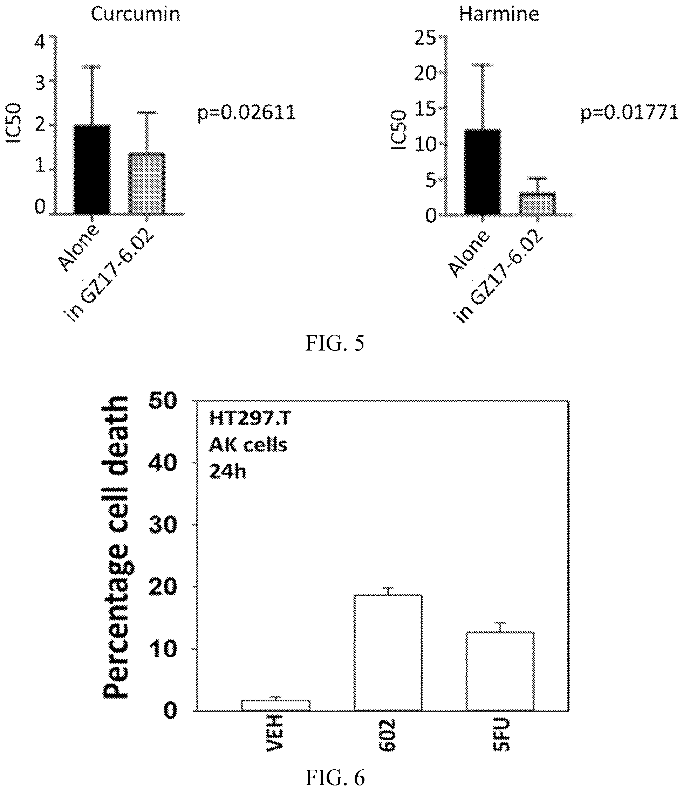

Additional internal studies have also demonstrated GZ17-6.02's ability to effect actinic keratosis cell lines. In a preclinical in vitro model, GZ17-6.02 was observed to have an IC50 of 5.07 ug/ml in the actinic keratosis cell line HaCaT. EGF-stimulated HaCaT cells were treated with GZ17-6.02, or individual quantities of harmine, curcumin, and isovanillin. GZ17-6.02 at this concentration contained Isovanillin 28.26 uM, Harmine 3.11 uM, and Curcumin 1.38 uM. The mean IC50 values for GZ17-6.02, curcumin, and harmine were 5.07 ug/mL, 0.7736 ug/mL, and 2.548 ug/mL, respectively. The mean IC50 values of curcumin and harmine when part of the combined formulation (as derived from the IC of GZ17-6.02) were 1.38 uM and 3.11 uM, respectively. As shown in Fig. 5, comparison of the mean IC50 values for both curcumin and harmine in isolation and derived from the IC50 of GZ17-6.02 demonstrate that the IC of each compound were significantly lower when part of the combined 6.02 formulation.

Interestingly, as shown in Fig. 6, 5-Fluorouracil (5FU) was observed to have an IC50 of 4.80 uM while diclofenac was observed to an IC50 of 601.7 uM under the same experimental methods. Multiple cell proliferation and survival signalling pathways are affected by GZ17-6.02 in actinic keratosis. DNA damage caused by GZ17-6.02 induces autophagy leading to cell death and subsequent changes in apoptotic, proliferation, survival, and other cellular signalling pathways. To investigate the mechanisms by which these pathways are affected, GZ17-6.02 was administered to the actinic keratosis cell line HT297.T in vitro and changes in signalling within various pathways were measured 6 hours post administration with cell death measured 24 hours post administration. It was observed that increased autophagy led to stimulation of apoptosis and decreased expression of survival and proliferation proteins. These processes lead to increased percentage cell death of actinic keratosis cells when compared against vehicle control (VEH), antimetabolite 5-Fluorouracil (5FU). The amount of GZ17-6.02 fixed ratio tested was based upon a 2 uM concentration of Curcumin (37.23 uM of Isovanillin, 4.51 uM of Harmine, 2 uM of Curcumin). 5-Fluorouracil was tested at a concentration of 50 uM.

### Mechanism of Action

GZ17-6.02 has demonstrated an ability to kill actinic keratosis cells through multiple convergent mechanisms. *In vitro* studies have demonstrated these mechanisms include such process as decreased cellular survival signalling via inhibition of the AMP-dependent protein kinase (AMPK)/Mammalian target of rapamycin (mTOR) signalling cascade, Epidermal Growth Factor Receptor (EGFR) signalling, and various effects on additional canonical signalling pathways.

GZ17-6.02 caused DNA damage activates Ataxia-telangiectasia mutated (ATM), which is a component of the DNA damage response and also a response to increased reactive oxygen species levels in cells. Downstream of ATM, GZ17-6.02 activates AMPK. Activated AMPK then inactivates mTOR Complex 1 (mTORC1) and mTOR Complex 2 (mTORC2). mTORC1 controls a wide range of cellular process that promote cellular proliferation while mTORC2 promotes cell growth through activation of various protein kinases. This inactivation of mTORC1 and mTORC2 leads to decreased cellular proliferation and activates a cascade leading to the formation of autophagosomes which is then followed by autophagic flux (autophagy).

Epidermal Growth Factor Receptor (EGFR) is a tyrosine kinase whose function is required for normal skin development and homeostasis. Dysregulation of EGFR signalling results in cellular hyper-proliferation and defects in differentiation, leading to impaired wound healing, the development of psoriasis-like lesions, structural and function defects of hair follicles and tumorigenesis. GZ17-6.02 decreased EGFR signalling in the actinic keratosis cell line HT297.T. This effect was observed in the entire ERBB receptor family (ERBB 1-4) and is congruent with published and unpublished data of GZ17-6.02. In unpublished preclinical studies, GZ17-6.02 decreased the effect of EGFR in colorectal, and melanoma cell lines.

GZ17-6.02 also causes an endoplasmic reticulum stress response, increasing Eukaryotic translation initiation factor 2A (eIF2a) phosphorylation, which reduces bulk protein translation by ~95% but up-regulates the protein levels of the other ~5%, including the levels of the proteins Beclin1 and ATG5 which were essential for autophagosome formation. GZ17-6.02 also increased the protein levels of the death receptor CD95. The increase in autophagy was partially causal in causing cell death as was signalling by the death receptor CD95.

### Enhanced stability formulations

Formulations with enhanced stability and delivery of the active agents are contemplated here. The formulation is water-based and comprises at least one monoglyceride of a fatty acid. The crystalline monoglyceride can be added in the amount from about 10 to about 35 % by weight, preferably from about 15 to about 30% by weight, more preferably from about 18 to about 28% by weight, depending upon the desired viscosity of the composition. Preferably the crystalline monoglyceride is a monoglyceride of a fatty acid chain length of C8 to C16. The most preferred monoglycerides are saturated monoglycerides with carbon chain lengths from 8 to 16 while the more preferred monoglycerides have carbon chain lengths of 10 to 14 carbon atoms, even more preferably 12 to 14 carbon atoms. Preferred examples of the crystalline monoglycerides include glycerol monolaurate (C12), glycerol monomyristate (C14), and mixtures thereof. The amount of and the ratio between C12 and C14 can be varied depending on the desired viscosity of the final product. For example, the weight ratio of C12:C14 may vary from about 1:10 to 1:2, preferably 1:4 to 1:2, with about 1:3 being particularly preferred. It will be appreciated that lower amounts of the monoglyceride can be used for different formulations, e.g., as low as 0.5% for a lotion instead of a cream.

The invented cream formulation can comprise a solvent or dispersion media, advantageously water. The water can make up the balance of the composition. The composition can also include pH and tonicity regulating components as well.

Other pharmaceutically acceptable components may be added to the composition. Buffering agents, pH and tonicity regulating compounds such as, but not limited to, phosphoric acid, citric acid, lactic acid sodium hydroxide, and the like can be introduced. The formulations are typically adjusted to a pH of between pH 3.5 to 6, preferably from about 4 to about 5.5, even more preferably from about 4 to about 5

Additional stabilizers such as pyrophosphate and sesquestrants such as but not limited to EDTA and phosphonic acids are also possible to incorporate into the composition. Physical stabilisers, against separation of water, such as nonionic surfactants of high HLB for the purpose of improving texture and cosmetic feel, and thickeners such as cellulose derivatives (e.g., hydroxypropyl methylcellulose), or polyacrylic acid derivatives may also be added to the composition to improve its properties. Traditional dermatological agents such as glycerol and propylene glycol may be added to enhance cosmetic properties. Surface modifying compounds such as surfactants and co-solvents, for example, dispersion of crystals or modification of surface charges, may be added, such as EDTA, span 20, polysorbate 80, polyoxyethylene (100) stearate, and the like. Nonlimiting examples of useful acids are citric acid and lactic acid. Nonlimiting examples of such surfactants are esters of fatty acids and alcohols or saccharides of polar nature. In particular, the composition of the dispersion media, which is typically predominantly water-based, can be modified by introducing polar solvents that are miscible with water. Nonlimiting examples of such solvents are: glycerol, propylene glycol, dipropylene glycol, polyethylene glycol, lactic acid and citric acid. Other nonlimiting examples are solutions of polar compounds such as urea, saccharides and acids in water and esters of lactic acid and citric acid.

Exemplary components and ranges for the lipid crystalline formulation are provided in the table below.

| **Component** | **Exemplary range % (w/w)** | **Alternate range % (w/w)** | **Alternate range % (w/w)** | **Alternate range % (w/w)** |
|---|---|---|---|---|
| **Active Agent Total** | 0.1-25 | 0.5-20 | 1-15 | 3-9 |
| Isovanillin | 0.05-21.25 | 0.4-15 | 0.7-12 | 2-7 |
| Harmine | 0.007-12.5 | 0.05-10 | 0.1-7.5 | 0.4-1.2 |
| Curcumin | 0.005-10 | 0.04-8 | 0.05-5 | 0.3-1.2 |

Based upon the total weight of the composition than as 100% by weight.

| **Component** | **Exemplary range % (w/w)** | **Alternative range % (w/w)** | **Alternative range % (w/w)** |
|---|---|---|---|
| **Carrier Formulation** | | | |
| Glycerolmonomyristate | 0.5-25 | 5-21 | 14-19 |
| Glycerolmonolaurate | 0.5-10 | 2-7.5 | 3-6.5 |
| Hydroxypropyl methylcellulose | 0-2 | 0-1.5 | 0-1 |
| Polyoxyethylene (100) stearate | 0-3 | 0-2 | 0-1.5 |
| Glycerol | 0-30 | 0-20 | 0-10 |
| Polysorbate 80 | 0-3 | 0-2 | 0-1.5 |
| Citric acid | 0-1 | 0.1-1 | 0.3-0.8 |
| NaOH | 0-1 | 0.01-0.5 | 0.1-0.3 |
| Water to 100 % | balance | 65-85 | 68-75 |

Based upon the total weight of the composition than as 100% by weight.

The active ingredients are dispersed in the cream and the monoglycerides (glycerol monomyristate and glycerol monolaurate) forms lipid crystals, preferably as a three-dimensional matrix or crystalline network in which the active ingredients are dispersed and/or encapsulated, and thus stabilized. Preferably, the lipid crystals are in the beta prime form, which is a more stable polymorph of the crystalline forms having a higher melting point. Thus, the monoglyceride cream will be shelf-stable at ambient conditions preferably having a somewhat self-supporting crystalline structure (i.e., it can retain its shape even without a support such as a container), but will melt at skin temperatures to release the stabilized active ingredients that were previously dispersed in the and stabilized by the lipid crystal structure. The monoglyceride cream, having an aqueous base, can be applied directly or can be formulated in other forms, such as dispersion of the stabilized cream into a number of compatible carrier vehicles to yield ointments, pastes, lotions, gels, semi-solid sticks, and the like.

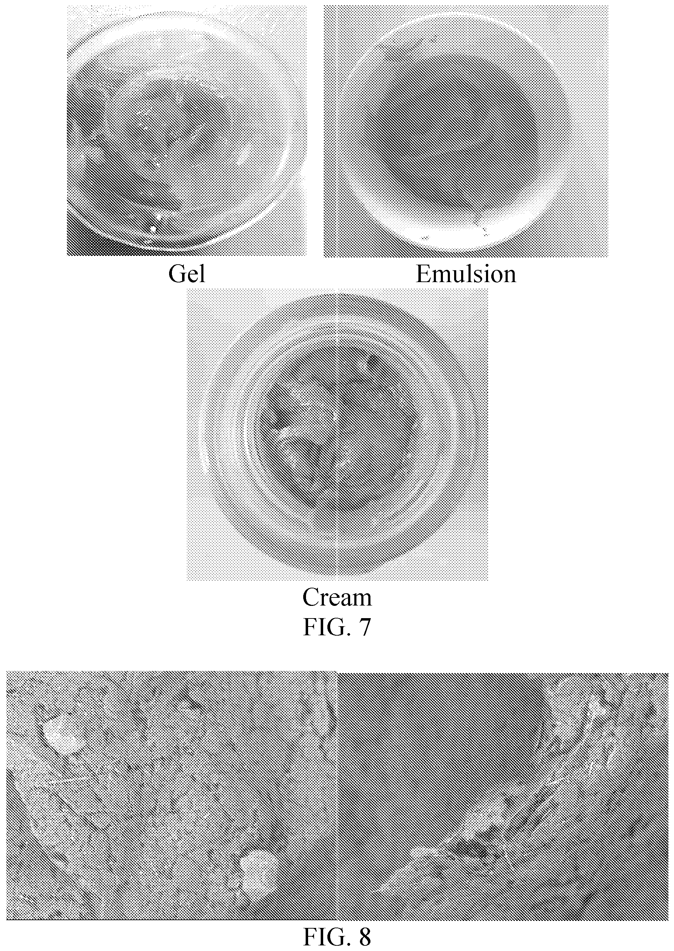

In one or more embodiments, the cream is orange and shimmering in appearance as shown in Fig. 7. In a particular embodiment, the formulation is a topical (cutaneous) cream comprising (consisting essentially or even consisting of) 3% (w/w) of the active drug compound GZ17-6.02, i.e., comprising (consisting essentially or even consisting of) about 2.3% Isovanillin component, about 0.39% Harmine component, and about 0.3% Curcumin component. In a particular embodiment, the formulation is a topical (cutaneous) cream comprising (consisting essentially or even consisting of) 6% (w/w) of the active drug compound GZ17-6.02, i.e., comprising (consisting essentially or even consisting of) about 4.6% Isovanillin component, about 0.78% Harmine component, and about 0.60% Curcumin component. In a particular embodiment, the formulation is a topical (cutaneous) cream comprising (consisting essentially or even consisting of) 9% (w/w) of the active drug compound GZ17-6.02, i.e., comprising (consisting essentially or even consisting of) about 6.93% Isovanillin component, about 1.17% Harmine component, and about 0.9% Curcumin component. In one or more embodiments, the relative amounts of the Harmine component and Curcumin component may be switched. That is, the 3% formulation may comprise about 0.3% Harmine component and about 0.39% Curcumin component, the 6% formulation may comprise about 0.6% Harmine component and about 0.78% Curcumin component, and the 9% formulation may comprise about 0.9% Harmine component and about 1.17% Curcumin component. Thus, the relative amounts of the Isovanillin component, Harmine component, and Curcumin component in a preferred formulation are approximately 77% w/w Isovanillin component (+/-5%), about 13% w/w Harmine component (+/-5%), and about 10% w/w Curcumin component (+/-5%), based upon the total weight of the three actives taken as 100% w/w. Alternative formulations are contemplated herein, provided that the Isovanillin component remains the preponderant active agent (>50% w/w) with the Harmine component and Curcumin component making up the balance.

In the inventive formulation all three active agents are combined to a chemically and physically stable formulation. This has been achieved by using a cream formulation that contains crystals of monoglycerides that yield a solid or semi-solid (i.e., self-supporting) matrix at ambient temperature and fluid and/or fluid crystalline at skin temperature. The cream formulation will then be semisolid at ambient temperature (~25°C +/- 2°C) and fluid at skin/body temperature (~30-37°C +/- 2°C). This formulation comprises acylmonoglycerides that are transferred to solid crystals during manufacture. When a chemical substance is in solid form the chemical stability is high since the reactions causing poor stability of the active require an active agent in dissolved state, otherwise no reactions can occur. Another aspect is the transport, diffusion, of reactants and other participants in the degradation reactions. When the diffusion of these compounds can be kept at a minimum, the degradation rate will be kept low.

The inventive formulation is manufactured using crystalline lipids which are defined by a continuous repeated structure in three dimensions but the nature of the repetition may not be the same in all directions. The crystals may contain bilayers of water and lipid creating a repeated structure of water and lipid layers in one direction and lipid crystals in two directions. A way to detect crystallinity is by electron microscope. For example, a definition of a lipid lamellar crystal is a solid crystal with three-dimensional continuity having the same repeated cells in two dimensions, but a different one in the third dimension, which can be established by wide angle X-ray. The crystallinity of monoglycerides in the compositions can be determined by differential scanning calorimetry (DSC). The transfer to solid liquid crystals is exothermic and gives rise to a release of energy. This can be determined by a scanning calorimeter. The reason for using more than one monoglyceride is that the monoglycerides are mutually soluble in each other and the crystals generated form a mixture that is lower in energy than from a single monoglyceride.

There are several advantages associated with the use of (solid) lipid crystals. Since the crystalline state in general is the lowest energetic state very little will happen with the structure during storage. In contrast to emulsions and liquid crystals which are changing over time by crystallization, sedimentation or coalescence, the solid lipid crystalline structures do not change over time in a pharmaceutical perspective, three to four years. Stable constituents and structures are regarded as a large advantage in the development of pharmaceutical products.

Another important embodiment of the invention is the ability of the cream to melt at body temperature when applied to the skin. When lipid crystals melt, a liquid crystalline structure will be formed. The content of the cream, active agents, pH modifying agents, etc. can then be released to the tissue and the product can exert its therapeutic effect. Such cream should preferably melt at a temperature of 25 to 37°C more preferable at 30 to 37°C and most preferably at 32 to 37°C.

In a typical but non-limiting procedure the solid lipid crystals are manufactured by heating the lipid(s) in water to 70 to 75°C to melt the lipids followed by slow cooling to crystallization temperature to solidify the lamellar crystals. The monoglycerides crystallize at 35 to 32°C and since the reaction is exogen, heat will develop. The active agents can be added prior to, during or after heating and even after cooling. It should however be noted that the stability of the active compounds could be impacted by being exposed to heating to over 70 °C. The cooled dispersion of crystals in water can then be diluted, if required, and mixed with other agents prior to packing. Non-limiting examples of such agents are pH modifiers, solvents, viscosity enhancers, humectants, emollients, chemical and physical stabilizers and preservatives. For the purpose of changing viscosity and cosmetic feel, emollients, humectants and viscosity increasing compounds can be added to the formulation. This can be determined by a person skilled in the art.

The crystalline monoglyceride formulation can be combined with a standard cream, lotion, suspension, emulsion, or solution formulations, and the total amount of crystalline lipid in that formulation can be estimated by a person skilled in the art. The medically or veterinary active ingredient or ingredients suitable in this formulation are isovanillin, harmine, curcumin or derivative thereof.

In some embodiments, the preferred amounts of actives in the formulation are based on pharmacological argumentation and therapeutic efficacy as balanced with formulation compatibility. Difficulties encountered in formulating compositions comprising two or more actives having different properties, including solid vs. liquid actives, solubility of the actives in the carrier formulation, hydrophobicity/hydrophilicity, and the like. Active agents can also aggregate or clump together or degrade or precipitate out of the carrier. It will be appreciated that these issues can vary depending upon the particular combination of 2 or 3 active agents used, as well as the particular surfactants and other additives used. Advantageously, the innovative crystalline monoglyceride formulation addresses the unique needs of isovanillin, harmine, and curcumin, which have different properties, and allows them to be formulated and delivered together as a synergistic combination for a topical application.

One way of expressing the stability is that the three actives are in different states in the formulation depending on their solubility and polarity properties. Without wishing to be bound by theory, the curcumin is believed to be encapsulated inside the lipid crystals and has thus no contact with water enhancing the chemical stability of this compound. Isovanillin is mainly suspended as solid particles in the water phase; however, it is believed to be present in both the lipid phase and the aqueous phase. Isovanillin's solubility in water is observed to have pH dependence and the solid state of isovanillin in water and the aqueous phase may explain the stability in this formulation. Harmine is partially soluble in the water phase but is still stable. The beta prim crystals of monoglycerides have earlier decreased reactions mediated by free radicals (autooxidation of peroxides) and this may explain the stability. It will be appreciated that a formulation comprising three different active agents with three different solubility properties being stabilized in a single formulation represents a significant advancement in the formulation and topical delivery of these synergistic combinations of active agents.

Dosage levels administered to mammalian subjects using the combination products of the invention are quite variable owing to factors such as the subject's age, subject's physical condition, the type of condition(s) being treated (e.g., specific cancer(s)), and the severity of the conditions. Determination of proper dosage levels can readily be determined by those skilled in the art. The combination products of the invention are particularly useful for the treatment of mammalian subject suffering from various cutaneous conditions, including skin cancers and related conditions such as actinic keratosis, as well as conditions where cutaneous or topical delivery of the synergistic combination of active agents in a stabilized form would be beneficial. In one or more embodiments, the curcumin is present at a level of from about 5-40% by weight, the harmine is present at a level of from about 7-50% by weight, the isovanillin is present at a level of from about 25-85% by weight, all of the foregoing based upon the total weight of the three actives (e.g., curcumin, harmine, and isovanillin), taken as 100% by weight. In one or more embodiments, a weight ratio of isovanillin:harmine:curcumin in the compositions is approximately 0.1-25:0.1-5:0.1-5. Preferably, the weight ratio of isovanillin:harmine:curcumin in the compositions is approximately 77:13:10 (+/-5). In terms of amounts, the isovanillin being is at a level of from about 25-85% by weight, the harmine is present at a level of from about 7-50% by weight, and the curcumin being is at a level of from about 5-40% by weight, all based on the total weight of the isovanillin, harmine, and curcumin taken as 100% by weight. Moreover, in certain embodiments the isovanillin is present at a level of at least about three times greater than each of the harmine and curcumin. These compositions provide a synergistic and/or adjuvant effect when used in combination.

The formulations may include one or more additional active or inactive pharmaceutically-acceptable ingredients. As used herein, the term "pharmaceutically-acceptable" means not biologically or otherwise undesirable, in that it can be administered to a subject without excessive toxicity, irritation, or allergic response, and does not cause any undesirable biological effects or interact in a deleterious manner with any of the other components of the composition in which it is contained.

Formulations herein are useful for medical and/or veterinarian use. The formulations can be administered via the topical or intracavital route. It will be appreciated that therapeutic and prophylactic methods described herein are applicable to humans as well as any suitable animal, including, without limitation, dogs, cats, and other pets or captive animals (e.g., zoo animals, research subjects), as well as, rodents, primates, horses, cattle, pigs, etc. The methods can be also applied for clinical research and/or study.

In one or more embodiments, the formulation may be administered to a subject (e.g., mammal) in need thereof. The subject may be suffering from, suspected of having, or at risk of exposure to or developing a condition which may be treated or ameliorated by the active ingredients. In one or more embodiments, a therapeutically effective amount of the formulation is administered to the subject in order to treat or ameliorate the condition in the subject.

In another embodiment, the methods comprise (consist essentially or even consist of) administering a therapeutically or prophylactically effective amount of the above-described formulation(s) to a subject in need thereof. Such individual may be symptomatic or asymptomatic. Thus, "therapeutic" use of the formulation(s) refers to processes that are intended to produce a beneficial change in an existing condition of a subject, such as by reducing the severity of the clinical symptoms and/or effects of the condition, and/or reducing the duration of the condition/symptoms/effects. Likewise, "prophylactic" use refers to processes that are intended to inhibit or ameliorate the effects of a future condition to which a subject may be exposed. In some cases, the formulation(s) may prevent the development of observable morbidity from the condition (i.e., near 100% prevention). In other cases, the compounds may only partially prevent and/or lessen the extent of morbidity due to the condition (i.e., reduce the severity of the symptoms and/or effects of the condition, and/or reduce the duration of the condition/symptoms/effects). In either case, the formulation(s) are still considered to "prevent" the condition.

### Local Absorption

Epidermal accumulation is significant due to the desired site of drug action on keratinocytes in actinic keratosis. Importantly, all three active pharmaceutical ingredients have shown ability to penetrate the epidermis and dermis in preclinical models. After topical application (in flow through diffusion cells, In-line cells from PermeGear Inc, Hellertown PA, USAl) isovanillin, harmine, and curcumin accumulate with the epidermis and, to a lesser degree, dermis.

**Table. Amount adsorbed into dermis (µg/g) and epidermis (µg/g) after application of 6 % GZ17-6.02 cream**

| Substance | Dermis | Epidermis |
|---|---|---|
| Isovanillin | 29.4 ± 19.3 | 181.2 ± 118.3 |
| Harmine | 38.9 ± 24.4 | 585.5 ± 227.8 |
| Curcumin | 0.40 ± 0.23 | 3.82 ± 1.88 |

These data, when analyzed in tandem with IC50 efficacy data, provide rationale that sufficient amounts of each active pharmaceutical ingredient are absorbed into the appropriate tissue to allow for biological effect.

### Systemic Absorption

Thus far in the phase 1 clinical trial (NCT03775525), patients taking an oral formulation of GZ17-6.02 have seen very little toxicity based on systemic exposure. This was based upon a systemic total daily dose of 750mg. The proposed topical formulation of GZ 17-6.02 is a 6% cream. In the interest of being cautious, if one were to assume 100% penetration and absorption of drug, one to two grams (exaggerated dose) of cream applied daily would be 8% (60 mg) to 16% (120 mg) of the systemic exposure in the oncology trial. Pre-clinical studies have demonstrated minimal systemic dermal absorption of any of the three active pharmaceutical ingredients. After 24 h of exposure only 208 µg/cm² Isovanillin, 4 µg/cm² Harmine and no curcumin was found to permeate the skin membranes. According to this in vitro study the systemic adsorption would be very small. Typically, an area of 25 cm² is treated in a clinical situation. According to presented in vitro study this would give rise to a systemic adsorption of 5.2 mg (25 cm² * 208 µg/cm²) Isovanillin and 0.1 mg (25 cm² * 4 µg/cm²) Harmine which is significantly lower than for previous oral study where a dose of 750 mg was administered.

**Table. Cumulative amount permeated (µg/cm²) after application of 6% GZ17-6.02 cream batch. Mean ± SD are given.**

| **Substance** | **2 h** | **4 h** | **6 h** | **8 h** | **24 h** |
|---|---|---|---|---|---|
| Isovanillin | 35.0 ± 20.7 | 103.4 ± 47.6 | 169.3 ± 66.1 | 209.5 ± 74.4 | 207.9 ± 95.2 |
| Harmine | 0.04 ± 0.06 | 0.20 ± 0.22 | 0.49 ± 0.43 | 0.85 ± 0.68 | 3.88 ± 2.14 |
| Curcumin | n.d. | n.d. | n.d. | n.d. | n.d. |

It has been the inventors' experience that systemic oral doses much larger than doses achievable by topical application have been shown to be safe and well tolerated by human subjects observed while part of a protocol-controlled phase 1 clinical trial. To date, all observed adverse effects appear to be dose dependent and transient. These adverse effects were observed at dosage levels many times higher than topical dosages.

Based upon the data reported here and the underlying mechanisms of action, the 3-component topical cream in the stabilized formulation can be used to treat a variety of conditions, including, but not limited to, Pre-malignant conditions: Actinic keratosis; Malignant conditions : Lymphoma (particularly mycosis fungoides and other primary cutaneous lymphomas, including, but not limited to B and T Cell lymphomas); basal cell carcinoma, squamous cell carcinoma and squamous cell carcinoma in situ, lentigo maligna and melanoma in situ, melanoma, Merkel Cell Carcinoma; Other lymphoproliferative conditions including but not limited to: Pityriasis lichenoides et variliformis acuta, pityriasis lichenoides chronica, cutaneous lymphoid hyperplasia, angiolymphoid hyperplasia with eosinophilia (a lot of these exist on a spectrum and are somewhat considered to be on a continuum with cutaneous lymphomas, but at the time of diagnosis are not malignant); Genetic conditions: tuberous sclerosis, neurofibromatosis types 1 and 2; Benign tumors: including but not limited to: seborrheic keratosis, neurofibromas, angiokeratomas, angiofibromas/fibrous papule, sebaceous adenoma, sebaceous hyplerplasia, melanocytic nevi, and other benign adnexal skin tumors; Metabolic: acanthosis nigricans; Inflammatory conditions: Psoriasis, atopic dermatitis, lichen planus, seborrheic dermatitis, lichen striatus, allergic contact dermatitis, irritant contact dermatitis, intertrigo, stasis dermatitis; Infectious conditions: Viral: Verrucae (all types including verruca vulgaris and condyloma acuminata), herpes simplex 1 and 2, molluscum contagiosum; bacterial: staphylococcus aureus, streptococcal infections; Fungal and yeast: tinea (superficial dermatophyte infections), candida. Prototozoal and parasitic: Leishmaniaisis; Neutrophilic conditions: pyoderma gangrenosum; dermatitis herpetiformis; Other skin conditions: infectious and non-infectious folliculitis; pruritus, prurigo nodularis, prurigo; and Autoimmune blistering conditions: Bullous pemphigoid, pemphigus group.

The above-described formulation(s) may be administered or applied to a subject in any convenient manner for topical application, such as by oral, rectal, nasal, ophthalmic, cutaneous (e.g., dermal patch). The dosage forms of the invention may be in the form of liquids, gels, suspensions, solutions, emulsions, lotions, creams, salves, ointments, or sprays. Moreover, therapeutically effective amounts of the formulation(s) of the invention may be co-administered with other active agents, where the two products are administered substantially simultaneously or in any sequential manner.

As noted, levels of dosing to subjects of the above-described formulation(s) are quite variable owing to factors such as the patient's age, patient's physical condition, and the severity of the disease. In general, the formulation with will be applied from 1 to 5 times per day to the affected area, depending upon the active agent concentration in the formulation used, the extent of the condition, and body weight of the patient. A person of skill in the art can determine the appropriate dosing regimen to deliver a therapeutically effective amount of the active compounds to the treated area. As noted in the data, high levels of the actives, although not required, are well tolerated with no signs of toxicity. Moreover, due to the synergistic nature of the combination, the combined formulation allows the use of reduced quantities of the actives while still achieving favorable IC50 values as compared to individual ingredients.

Additional advantages of the various embodiments of the invention will be apparent to those skilled in the art upon review of the disclosure herein and the working examples below. It will be appreciated that the various embodiments described herein are not necessarily mutually exclusive unless otherwise indicated herein. For example, a feature described or depicted in one embodiment may also be included in other embodiments but is not necessarily included. Thus, the present invention encompasses a variety of combinations and/or integrations of the specific embodiments described herein.

As used herein, the phrase "and/or," when used in a list of two or more items, means that any one of the listed items can be employed by itself or any combination of two or more of the listed items can be employed. For example, if a composition is described as containing or excluding components A, B, and/or C, the composition can contain or exclude A alone; B alone; C alone; A and B in combination; A and C in combination; B and C in combination; or A, B, and C in combination.

The present description also uses numerical ranges to quantify certain parameters relating to various embodiments of the invention. It should be understood that when numerical ranges are provided, such ranges are to be construed as providing literal support for claim limitations that only recite the lower value of the range as well as claim limitations that only recite the upper value of the range. For example, a disclosed numerical range of about 10 to about 100 provides literal support for a claim reciting "greater than about 10" (with no upper bounds) and a claim reciting "less than about 100" (with no lower bounds).

### EXAMPLES

The following examples set forth methods in accordance with the invention. It is to be understood, however, that these examples are provided by way of illustration.

### EXAMPLE

Manufacture of a gel containing isovanillin, harmine, and curcumin

### Gel.

| **Component** | **% (w/w)** | **% (w/w)** |
|---|---|---|
| Isovanillin | 4.62 | 6.16 |
| Harmine | 0.78 | 1.04 |
| Curcumin | 0.60 | 0.80 |
| PEG 400 | 39.0 | 39.0 |
| PEG 4000 | 13.0 | 13.0 |
| Lactic acid | 2.0 | 2.0 |
| Glycerol | 20.0 | 19.0 |
| Propylene glycol | 20.0 | 19.0 |

All components except isovanillin, harmine and curcumin were dissolved in a beaker by heating to 70 °C under constant stirring. Isovanillin, harmine and curcumin, were then added to the heated mixture and mixed until dissolved. The mixture was cooled to room temperature while stirring.

### EXAMPLE

### Manufacture of emulsions containing isovanillin, harmine, and curcumin

A first emulsion composition was prepared using the following ingredients and manufacturing method.

### Emulsion 1.

| **Component** | **% (w/w)** |
|---|---|
| **Polar phase (A):** | |
| Isovanillin | 4.62 |
| Harmine | 0.78 |
| Curcumin | 0.60 |
| PEG 400 | 30.0 |
| PEG 4000 | 10.0 |
| Lactic acid | 1.0 |
| Glycerol | 16.5 |
| Propylene glycol | 16.5 |

| **Lipid phase (B):** | |
|---|---|
| Sorbitan monostearate | 1.4 |
| Cetyl palmitate | 2.1 |
| Cetostearyl alkohol | 7.2 |
| 2-octyl-1-dodecanol | 9.3 |

Preparation of polar phase (A): All components except PEG 4000 were added into a container. The components were dissolved by stirring and mixed into a homogenous solution. The mixture was heated to approx. 75 °C during stirring. PEG 4000 was added to the warm mixture and dissolved.

Preparation of the lipid phase (B): All components were added into a container. The components were dissolved by stirring and mixing into a homogenous solution. The solution was heated during stirring to approx. 75 °C.

Preparation of emulsion o/p: The polar phase was slowly added to the lipid phase while stirring intensively at 75 °C. The mixture was homogenized intensively at temperature 75 °C until a homogenous emulsion was formed. The emulsion was cooled to room temperature during moderate stirring.

A second emulsion composition was using this same process, but with different lipid phase components. Different amounts of (totally 49-52% PEG) and types of Polyethylene glycol (PEG) have been tried. Always PEG 400 but in combination with high molecular PEGs to obtain a suitable viscosity e.g. PEG 6000, PEG 10000 and PEG 35000.

### Emulsion 2.

| **Component** | **% (w/w**) |
|---|---|
| **Polar phase (A)** | |
| Isovanillin | 5.2 |
| Harmine | 0.88 |
| Curcumin | 0.68 |
| PEG 400 | 32.28 |
| PEG 4000 | 10.76 |
| Lactic acid | 0.7 |
| Glycerol | 17 |
| Polypropylene glycol | 17 |

| **Lipid phase (B)** | |
|---|---|
| PEG 30 Dipolyhydroxystearate (cithrol) | 3.5 |
| Paraffin oil | 5.5 |
| Isohexadecane | 4 |
| Caprylic/capric triglyceride | 1 |
| Diethylhexyl Adipate | 1.5 |

### EXAMPLE

### Manufacture of a monoglyceride cream containing isovanillin, harmine and curcumin

In the Example, novel monoglyceride creams were prepared. The compositions can be characterized as a cream of suspended monoglyceride crystals, in which the active agents are stabilized. Comparison photographs are shown in Fig. 7.

### Composition 1 - Monoglyceride cream.

| **Component** | **Quantity % (w/w)** |
|---|---|
| Isovanillin | 4.62 |
| Harmine | 0.78 |
| Curcumin | 0.60 |
| Glyceryl monomyristate | 18.0 |
| Glyceryl monolaurate | 6.0 |
| Citric acid | 0.5 |
| Water | 69.5 |

To prepare the monoglyceride cream, citric acid was dissolved in water and the solution was heated to 75 °C. The lipids were then added and the mixture was stirred at 75 °C for about 15 minutes. The formulation was removed from the heat and allowed to cool to 40 to 50°C while stirring. The isovanillin, curcumin and harmine were then added, and the mixture was allowed to cool with constant stirring. Notably, for ease of manufacture, although preferred, this cooling step can be skipped and the active ingredients can be added (e.g., at 75 °C) without allowing the mixture to first cool.

The formulation crystallized at between 30 to 35 °C and an increase in temperature was observed due to the exothermic crystallization of the lipids. Once the exothermic rise was observed, the formulation cooled to ambient temperature.

### Composition Version 2 - Monoglyceride cream with polymer or surfactants

| **Component** | | **Quantity % (w/w)** | **Quantity % (w/w)** | **Quantity % (w/w)** | **Quantity % (w/w)** |
|---|---|---|---|---|---|
| Actives | | **6%** | **6%** | **6%** | **9%** |
| | Isovanillin | 4.62 | 4.62 | 4.62 | 6.93 |
| | Harmine | 0.78 | 0.78 | 0.78 | 0.90 |
| | Curcumin | 0.60 | 0.60 | 0.60 | 1.17 |
| | | | | | |
| Glycerolmonomyristate | | 15.00 | 15.00 | 15.00 | 18.00 |
| Glycerolmonolaurate | | 5.00 | 5.00 | 5.00 | 6.00 |
| Hydroxypropyl methylcellulose (HPMC) | | 1.0 | - | - | 1.0 |
| Polyoxyethylene (100) stearate | | - | 1.0 | | - |
| Polysorbate 80 | | - | - | 1.0 | - |
| Citric acid | | 0.50 | 0.50 | 0.50 | 0.5 |
| Water | | 72.5 | 72.5 | 72.5 | 65.5 |

To prepare the monoglyceride cream, citric acid was dissolved in water and the solution was heated to 75 °C. The HPMC and surfactants (Polyoxyethylene (100) stearate and polysorbate 80) were then added, along with the lipids. The mixture was stirred at 75 °C for about 15 minutes. The formulation was removed from the heat and allowed to cool to 60°C while stirring. The isovanillin, curcumin and harmine were then added, and the mixture was allowed to cool with constant stirring. Notably, for ease of manufacture, although preferred, this cooling step can be skipped and the active ingredients can be added (e.g., at 75 °C) without allowing the mixture to first cool.

The formulation crystallized at between 30 to 35 °C and an increase in temperature was observed due to the exothermic crystallization of the lipids. Once the exothermic rise was observed, the formulation cooled to ambient temperature.

### Composition Version 3 - Monoglyceride cream with surfactant, glycerol and preservatives

| **Ingredients** | | **Quantity % (w/w)** | **Quantity % (w/w)** |
|---|---|---|---|
| Actives | | 3% | 6% |
| | Isovanillin | 2.31 | 4.62 |
| | Harmine | 0.39 | 0.78 |
| | Curcumin | 0.30 | 0.60 |
| | | | |
| Glyceryl monolaurate | | 6.00 | 6.00 |
| Glyceryl monomyristate | | 18.00 | 18.00 |
| Polyoxyethylene (100) stearate | | 1.00 | 1.00 |
| Glycerol | | 5.00 | 5.00 |
| Citric acid, anhydrous | | 0.50 | 0.50 |
| Sodium hydroxide | | 0.06 | - |
| Methylparaben | | 0.10 | 0.10 |
| Phenoxyethanol | | 0.80 | 0.80 |
| Water | | 65.54 | 62.60 |

SEM images were made with SEM showing the surface on 6% (w/w) GZ17-6.02 monoglyceride cream. Sheets of lipid structures could be observed as well as crystalline material of the active ingredients. Representative images are shown in Fig. 8. The larger grain like particles are Isovanillin while the needle like structures likely are Harmine or Curcumin.

### EXAMPLE

### Stability testing

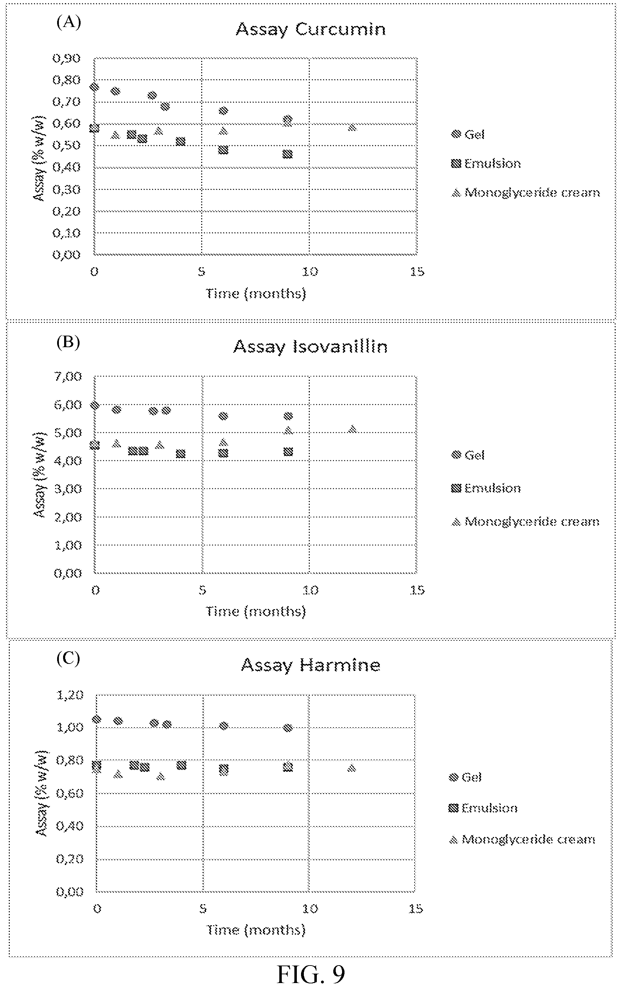

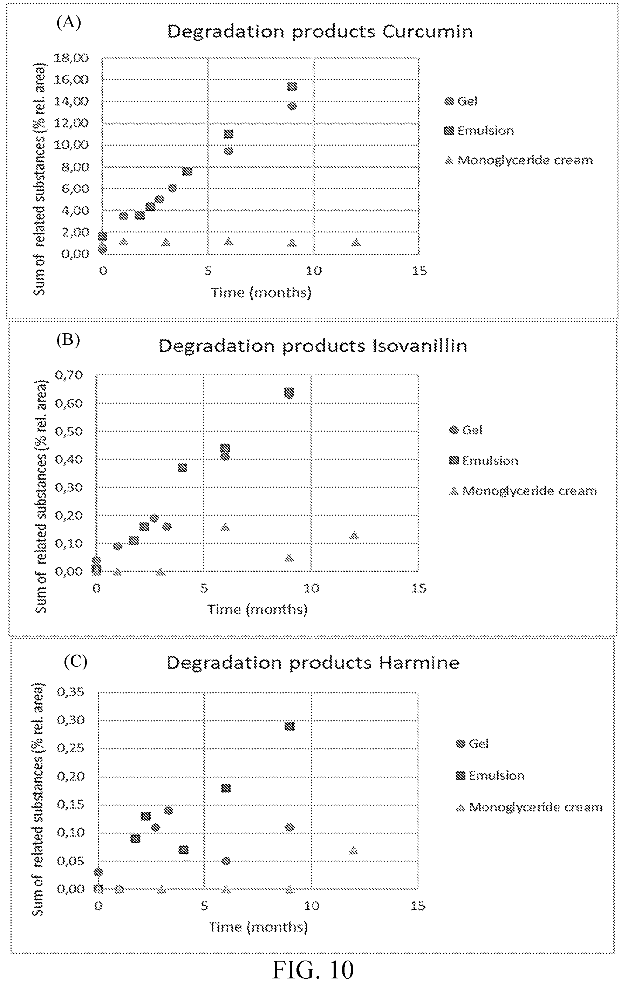

The stability of the different compositions was then tested. The gel and emulsion compositions were stored at 2-8°C, 25±2°C or at 40±2°C, and the cream composition was stored at 2-8°C, 25±2°C or at 30±2°C and checked for integrity of the active ingredients over a period of several months. Initial compositions had a pH of 4.1 to 4.5 and an orange or apricot appearance. Figs. 9 and 10 show graphs from the results from stability and degradation studies for the GZ21T formulation, stored at 25±2°C for up to 15 months.

### EXAMPLE

### In vitro penetration studies of monoglyceride cream

The product is a cutaneous cream containing 6 % G17-6.02, and has an orange, shimmering appearance, see composition in Table below. The drug compound G17-6.02 is comprised of the active ingredients in the relative proportions: 77% Isovanillin, 13% Harmine and 10% Curcumin. The formulation is filled into aluminium tubes to protect the formulation from light and to prevent evaporation.

| **Composition of cutaneous cream, 6 % G17-6.02** | | |
|---|---|---|
| **Ingredients** | **Quantity % (w/w)** | **Function** |
| GZ17-6.02: | 6.0 | Active |
| Isovanillin | 4.62 | |
| Harmine | 0.78 | |
| Curcumin | 0.60 | |
| Glyceryl monomyristate | 18.0 | Emollient /stabiliser |
| Glyceryl monolaurate | 6.0 | Emollient /stabiliser |
| Citric acid, anhydrous | 0.5 | Adjust pH |
| Water, purified | 69.5 | Solvent |
| **Total** | **100** | - |

| | | |
|---|---|---|
| * Complies with the USP-NF monograph "Mono- and Di-glycerides" | | |

The current formulation is based on crystalline lipids (glyceryl monolaurate and glyceryl monomyristate) which stabilizes the formulation. The lipids melt on the skin and can thereby deliver the active ingredient efficiently. Without wishing to be bound by theory, curcumin is believed to be embedded in the lipid crystals, whereas both Isovanillin and Harmine are partly soluble in water and is believed to be present in both the lipid phase and the aqueous phase.

By comparison, in one study, the solubility of the active ingredients in aqueous GZ17-6.02 solutions (not the lipid crystalline formulation) was first analyzed. The pH is adjusted to 4.3 with 0.5% (w/w) citric acid.

| **Active ingredient** | **pH** | **Solubility (% w/w) at 2-8 °C** | **Solubility (% w/w) at 25 °C** |
|---|---|---|---|
| Isovanillin | 4.3 | 0.27 | 0.57 |
| Harmine | 4.3 | > 1.56 | > 1.56 |
| Curcumin | 4.3 | n.d. | n.d. |
| Isovanillin | 7.0 | 0.18 | 0.28 |
| Harmine | 7.0 | 0.0014 | 0.0034 |
| Curcumin | 7.0 | n.d. | n.d. |

| | | | |
|---|---|---|---|
| n.d. = not detected | | | |

### EXAMPLE

### In-vitro permeation test (IVPT) of GZ17-6.02 monoglyceride cream through pig skin, reduced particle size of API

In this example, *in vitro* permeation (through pig skin) of 6% GZ17-6.02 MG creams with untreated and milled particles were compared (unmilled GZ17-6.02, milled GZ17-6.02, and milled Isovanillin + unmilled Harmine and Curcumin). No difference in permeation was found. Specifically, no significant differences between the evaluated formulations could be detected regarding the amount found in the receptor (Table 20) or the amount found in dermis (Table 21). For Isovanillin a depletion could be seen with decreased flux values after 6-8 hours while no depletion could be detected for Harmine and Curcumin.

When no treatment is done on the Isovanillin particles they are quite large while Curcumin consists of significantly smaller particles. The possibility to mill Isovanillin and/or GZ17-6.02 prior to formulation manufacture has been investigated.

### Results

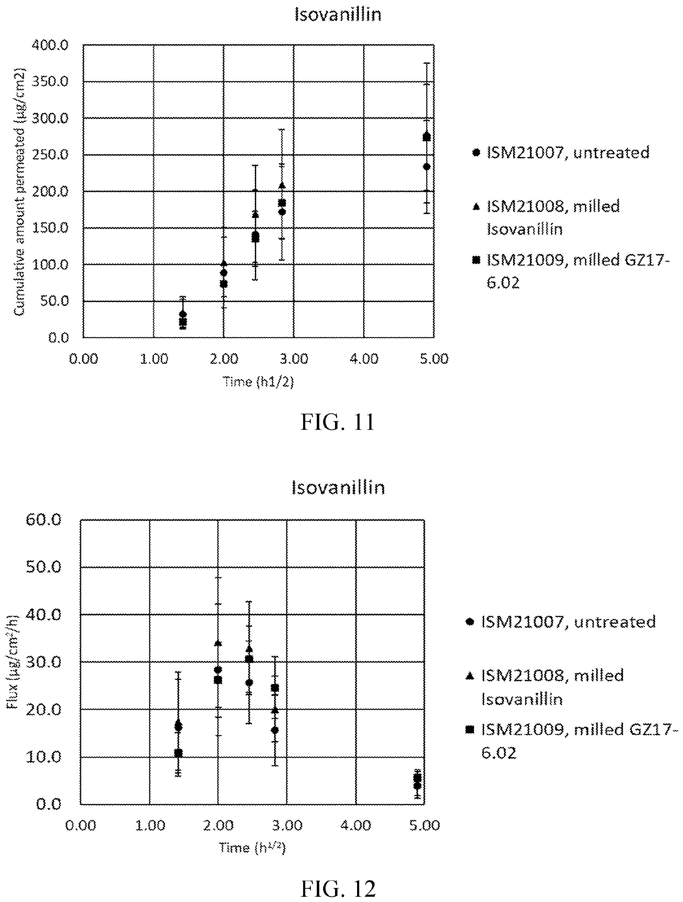

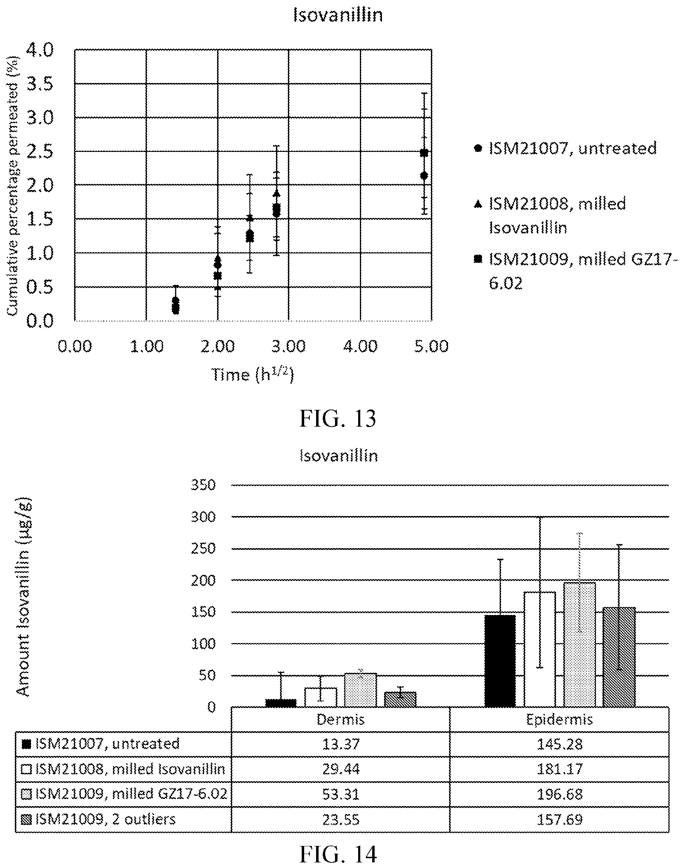

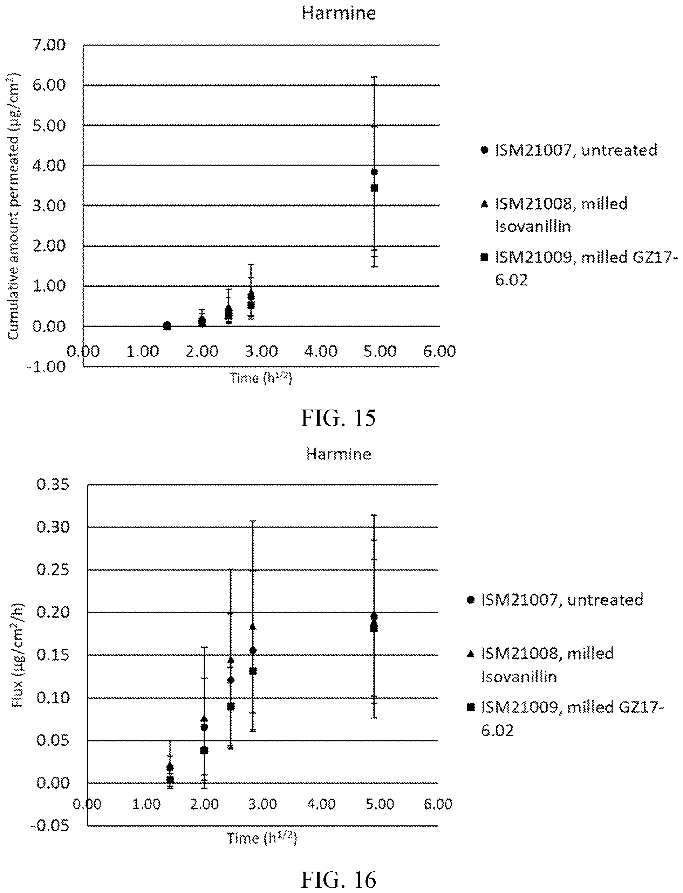

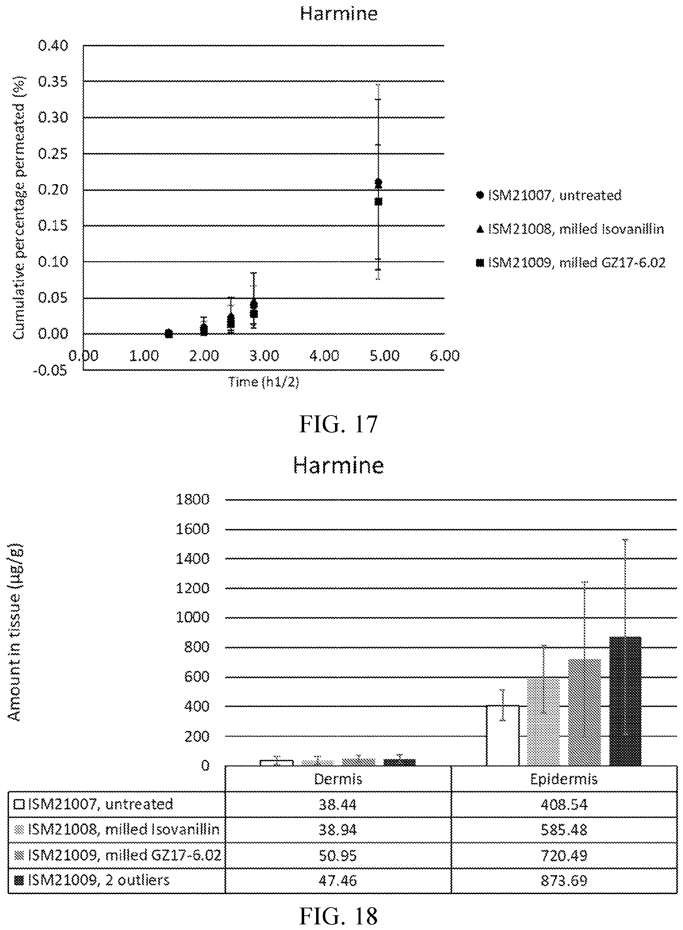

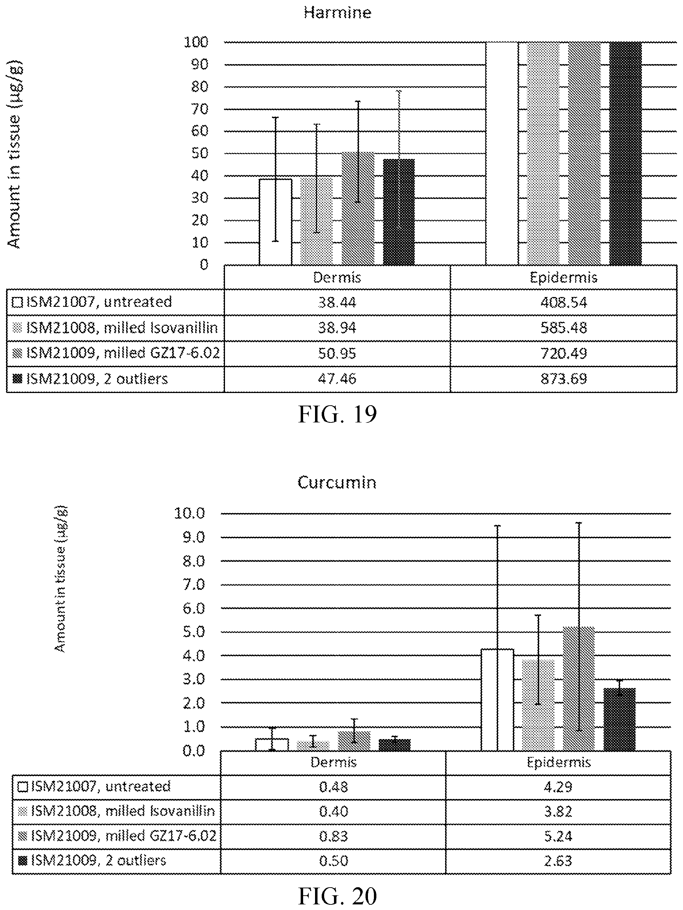

The results are shown in the tables below and also in Figs. 11-20.

### Cumulative amount of GZ17-6.02 substances permeated the skin into the receptor (µg/cm²)

| **Formulation** | **Isovanillin** | **Harmine** | **Curcumin** |
|---|---|---|---|
| ISM21007, untreated | 233.8 ± 63.3 | 3.84 ± 2.36 | n.d. |
| ISM21008, milled Isovanillin | 279.9 ± 95.2 | 3.88 ± 2.14 | n.d. |
| ISM21009, milled GZ17-6.02 | 273.6 ± 72.5 | 3.43 ± 1.53 | n.d. |

### Amount of GZ17-6.02 substances in dermis (µg/cm²)

| **Formulation** | **Isovanillin** | **Harmine** | **Curcumin** |
|---|---|---|---|
| ISM21007, untreated | 0.72 ± 0.37 | 2.03 ± 1.37 | 0.03 ± 0.02 |
| ISM21008, milled Isovanillin | 1.56 + 0.87 | 2.10 ± 1.25 | 0.02 ± 0.02 |
| ISM21009, milled GZ17-6.02 | 2.85 ± 2.16 | 2.77 ± 1.31 | 0.04 ± 0.02 |
| ISM21009, milled GZ17-6.02, 2 outliers | 1.29 ± 0.51 | 2.61± 1.76 | 0.03 ± 0.01 |

### Materials and methods

Three formulations were used in this experiment. One 6% GZ17-6.02 monoglyceride cream containing milled Isovanillin particles (ISM21007), one 6% GZ17-6.02 monoglyceride cream (ISM21008) containing milled GZ17-6.02 particles and one monoglyceride cream containing untreated GZ17-6.02 (ISM21009). An overview of the formulations is given in the table below.

### Composition of formulations investigated, % (w/w).

| **Ingredient** | **ISM21007 % (w/w)** | **ISM21008 % (w/w)** | **ISM21009 % (w/w)** |
|---|---|---|---|
| 1-Glycerolmonomyristate | 18.00 | 18.00 | 18.00 |
| 1-Glycerolmonolaurate | 6.00 | 6.00 | 6.00 |
| Isovanillin | 4.62 | - | - |
| Isovanillin (milled) | - | 4.62 | - |
| Curcumin | 0.60 | 0.60 | - |
| Harmine | 0.78 | 0.78 | - |
| GZ17-6.02 (milled) | - | - | 6.00 |
| Citric acid | 0.50 | 0.50 | 0.50 |
| Water to (69.5 %) | 100.00 | 100.00 | 100.00 |

### Receptor solution

PBS buffer with 2% (w/w) Brij will be used as receptor solution. The recipe of the PBS buffer solution is given in the table below.

| **Chemical** | **Internal ref number** | **% (w/w)** | **Composition for 1000 mL** |
|---|---|---|---|
| Sodium chloride | CH0013 | 0.765 | 7.65 g |
| di-Sodium hydrogen hosphate, dihydrate | CH0012 | 0.0908 | 0.908 g |
| Potassium dihydrogen phosphate | CH0009 | 0.021 | 0.210 g |
| Brij 020-SS-(RB) | CH0611 | 2.00 | 20.00 g |
| 1M NaOH | n.a. | to pH 7.4 | to pH 7.4 |
| HPLC water | n.a. | to volume | to 1000 mL |

### In vitro experiment

The equipment used is Bronaugh diffusion cells. Parameters of the experiment is described in tables below. Analyses were performed according to RP-HPLC assay with additional calibration standard solutions. Yellow colour was discovered under the membranes in cell 11 and 12 when the cells were dismounted. The occurred leakage likely occurred during washing of the cells and no effect could be seen in the receptor solutions. Hence results from all cells are reported for the receptor solution. For the amount found in the tissue values both with and without cell 11 and 12 are reported.

| **Equipment** | **Ref No.** | **Description** |
|---|---|---|
| Bronaugh cell equipment | Z99004 | One Retriever IV Fraction Collector, ISCO Inc., USA Two cell warmers and 14 in-line flow cells (diffusion area of 0.63 cm²), PermeGear ILC14 automated system, PermeGear Inc. USA |
| Water bath | Z06004 | Grant GD120; for temperatures in the range of 25-40°C |
| Peristaltic pump | Z04012 | Ismatec^{®} IPC |
| Tubing | | Tubing |

### Experimental parameters for in vitro release experiment

| **Parameter** | **Value/Interval** |
|---|---|
| Receptor phase composition | PBS with 2% (w/w) Brij |
| Light conditions | Exposure to light was decreased by covering the cells with Aluminum-foil |
| Treceptor phase | 34 °C giving a membrane temperature of 32.0°C |
| Membrane | Full thickness skin membranes from pig ear |
| Randomisation | Membranes will be randomized |
| Application interval | One application |
| Applied amount | 150 mg |
| Sampling timepoint | 2, 4, 6, 8, 24 h |
| Duration of study | 24 h |
| Occlusion | **No occlusion** |
| Analysis | RP-HPLC |
| Post experiment | Clean the membranes, separate epidermis and dermis and extract substances from the membranes. |

### In vitro release experiment

| **Cell** | **Formulation** | **Batch** | **Applied dose (mg)** |
|---|---|---|---|
| 1-4 | 6% GZ17-6.02 MG cream with untreated *GZ17-6.02* | ISM21007 | 150 mg |
| 5-9 | 6% GZ17-6.02 MG cream with milled Isovanillin | ISM21008 | 150 mg |
| 10-14* | 6% GZ17-6.02 MG cream with milled GZ17-6.02 | ISM21009 | 150 mg |

| | | | |
|---|---|---|---|
| *Cell 11-12 are possible outliers as leakage could be detected when cells were dismounted. No effect of this could be seen in the receptor solution but slightly larger levels were detected in dermis and epidermis for these cells. | | | |

### Post-experiment treatment

After the *in vitro* experiment was terminated each membrane was washed, dismounted and prepared according to the following procedure:
1. Wash the membrane six times with 0.5 ml PBS buffer and collect the PBS in the wash fraction tubes.
2. Dismount the cells and gently clean the membranes with PBS moisture tops. Collect the tops in the wash fraction tubes.
3. Gently separate stratum corneum from dermis by peeling the membrane and collect in a test tube.
4. Cut out the cell flange (the unexposed skin) with a 10 mm punch and collect the material in a test tube.
5. Cut the dermis into thin strips with a scalpel and collect the material in a test tube 1.5 ml.

### Analysis

Analysis of receptor solution and extractions from epidermis and dermis were performed by RP-HPLC.

### Receptor solution

Receptor solution were analyzed without any further treatment.

### Tissue extractions

Tissue samples were treated according to the following procedure:
1. Add 1.4 ml methanol/water (90/10 v/v) to extract isovanillin, harmine and curcumin from the membranes.
2. Vortex the sample and sonicate for 30 minutes in cold water (2-8°C add ice to the bath).
3. The samples should then be centrifuged at 14000 rpm and transferred to HPLC vial for analysis.

### Results and discussion

### Permeation of Isovanillin

### Amount in receptor solution

Quite large amount of Isovanillin permeates the skin membranes during the *in vitro* experiment as shown in the figures and tables. The permeation of Isovanillin shows a linear region in the cumulative amount permeate vs square root of time between 2 and 8 hours. Thereafter a deviation from the linear regime can be seen, which is explained by the decrease in flux seen after 6 hours. The deviation from the linear regime occurs after 6-8 hours when about 1.5 % of the applied dose has permeated the skin membrane and is likely caused by depletion from the formulation. Normally depletion is seen after about 30-50 % of the applied amount has permeated the membrane. The fast depletion seen here is reasonable as only small part of the added Isovanillin is dissolved and it can be assumed that only the dissolved Isovanillin is available for permeation. In addition, an infinite dose is applied and it is reasonable to believe that only the fraction closest to skin membrane is available to the skin.

A slight indication of lower permeation from the formulation with untreated Isovanillin (ISM21007), compared to the formulations with milled Isovanillin and GZ17-6.02 can be observed. However, the standard deviation, given as errors, are quite large and no significant differences can be observed between the formulations.

### Cumulative amount (µg/cm²) of Isovanillin permeated. Mean ± SD are given.

| **Formulation** | **2 /h** | **4 h** | **6 h** | **8 h** | **24 h** |
|---|---|---|---|---|---|
| ISM21007, 6% GZ17-6.02 MG cream, untreated | 32.4 ± 20.4 | 89.1 ± 48.0 | 140.5 ± 61.4 | 171.8 ± 65.6 | 233.8 ± 63.3 |
| ISM21008, 6% GZ17-6.02 MG cream, milled Isovanillin | 35.0 ± 20.7 | 103.4 ± 47.6 | 169.3 ± 66.1 | 209.5 ± 74.4 | 279.9 ± 95.2 |
| ISM21009, 6% GZ17-6.02 MG cream, milled GZ 17-6.02 | 21.7 ± 8.5 | 74.2 ± 24.2 | 135.4 ± 37.7 | 184.5 ± 48.9 | 273.6 ± 72.5 |

### Flux (µg/cm²/h) of Isovanillin permeated. Mean ± SD are given.

| **Formulation** | **2 h** | **4 h** | **6 h** | **8 h** | **24 h** |
|---|---|---|---|---|---|
| ISM21007, 6% GZ17-6.02 MG cream, untreated | 16.2 ± 10.2 | 28.3 ± 13.9 | 25.7 ± 8.7 | 15.7 ± 7.4 | 3.9 ± 2.5 |
| ISM21008, 6% GZ17-6.02 MG cream, milled Isovanillin | 17. 5 ± 10.4 | 34.2 ± 13.7 | 33.0 ± 9.7 | 20.1 ± 6.9 | 4.4 ± 2.6 |
| ISM21009, 6% GZ17-6.02 MG cream, milled GZ 17-6.02 | 10.9 ± 4.2 | 26.2 ± 7.9 | 30.6 ± 6.9 | 24.6 ± 6.5 | 5.6 ± 1.7 |

### Cumulative percentage of applied amount Isovanillin permeated (%). Mean ± SD are given.

| **Formulation** | **2 h** | **4 h** | **6 h** | **8 h** | **24 h** |
|---|---|---|---|---|---|
| ISM21007, 6% GZ17-6.02 MG cream, untreated | 0.30 ± 0.20 | 0.82 ± 0.46 | 1.29 ± 0.59 | 1.58 ± 0.62 | 2.14 ± 0.57 |
| ISM21008, 6% GZ17-6.02 MG cream, milled Isovanillin | 0.32 ± 0.20 | 0.93 ± 0.45 | 1.52 ± 0.63 | 1.88 ± 0.70 | 2.51 ± 0.85 |
| ISM21009, 6% GZ17-6.02 MG cream, milled GZ 17-6.02 | 0.20 + 0.07 | 0.67 ± 0.21 | 1.22 ± 0.33 | 1.67 ± 0.43 | 2.47 ± 0.65 |

### Amount in tissue

The amount of Isovanillin accumulated in epidermis and dermis are shown in the figures and tables. As can be seen a larger amount of Isovanillin is found in epidermis compared to dermis. One should however keep in mind that although the membranes were washed thoroughly, it cannot be guaranteed that all remaining of the formulation is removed. A slight tendency of increased accumulation in the tissue for the formulations which were more treated (ISM21008 with milled isovanillin and ISM 21009 with milled GZ17-6.02) can be seen although the deviation between the replicates are large giving rise to large standard deviations.

### Amount Isovanillin found in the tissue, Mean ± SD are given.

| **Formulation** | **Amount in Dermis (µg)** | **Amount in Dermis (µg/g)** | **Amount in Epidermis (µg)** | **Amount in Epidermis (µg/g)** |
|---|---|---|---|---|
| ISM210078, 6% GZ17-6.02 MG cream, untreated | 0.46 ± 0.24 | 13.37 ± 6.36 | 0.74 ± 0.40 | 145.28 ± 77.62 |
| ISM21008, 6% GZ17-6.02 MG cream, milled Isovanillin | 1.00 ± 0.56 | 29.44 ± 19.32 | 0.93 ± 0.61 | 181.17 ± 118.31 |
| ISM21009, 6% GZ17-6.02 MG cream, milled GZ17-6.02 | 1.82 ± 1.38 | 53.31 ± 41.46 | 1.01 ± 0.45 | 196.68 ± 87.76 |
| ISM21009, 6% GZ17-6.02 MG cream, milled GZ17-6.02, cell 11 and 12 outliers | 0.83 ± 0.33 | 23.55 ± 8.63 | 0.81 ± 0.50 | 157.69 ± 98.42 |

### Permeation of Harmine

### Amount in receptor solution

The amount of Harmine permeated the skin membranes can be seen in the figures and tables. After 8 hours it appears that a steady state flux is reached and the flux does not change much between 8 hours and 24 hours. No depletion from the formulation could be seen in agreement with low percentage of available Harmine permeated (0.2 % after 24 h) (Harmine is believed to be fully dissolved in the formulation).

No difference between the three formulations could be seen in the amount of Harmine which permeates the skin membranes.

### Cumulative amount (µg/cm²) of Harmine permeated. Mean ± SD are given.

| **Formulation** | **2 h** | **4 h** | **6 h** | **8 h** | **24 h** |
|---|---|---|---|---|---|
| ISM21007, 6% GZ17-6.02 MG cream, untreated | 0.04 ± 0.03 | 0.17 ± 0.14 | 0.41 ± 0.30 | 0.72 ± 0.48 | 3.84 ± 2.36 |
| ISM21008, 6% GZ17-6.02 MG cream, milled Isovanillin | 0.04 ± 0.06 | 0.20 ± 0.22 | 0.49 ± 0.43 | 0.85 ± 0.68 | 3.88 ± 2.14 |
| ISM21009, 6% GZ17-6.02 MG cream, milled *GZ17-6.02* | 0.01 ± 0.01 | 0.08 ± 0.08 | 0.26 ± 0.17 | 0.52 ± 0.27 | 3.43 ± 1.53 |

### Flux (µg/cm²/h) of Harmine permeated. Mean ± SD are given.

| **Formulation** | **2h** | **4 h** | **6 h** | **8 h** | **24 h** |
|---|---|---|---|---|---|
| ISM21007, 6% GZ17-6.02 MG cream, untreated | 0.02 ± 0.01 | 0.07 ± 0.06 | 0.12 ± 0.08 | 0.16 ± 0.09 | 0.20 ± 0.12 |
| ISM21008, 6% GZ17-6.02 MG cream, milled Isovanillin | 0.02 ± 0.03 | 0.08 ± 0.08 | 0.15 ± 0.11 | 0.18 ± 0.12 | 0.19 ± 0.10 |
| ISM21009, 6% GZ17-6.02 MG cream, milled GZ 17-6.02 | 0.00 ± 0.01 | 0.04 ± 0.03 | 0.09 ± 0.05 | 0.13 ± 0.05 | 0.18 ± 0.08 |

### Cumulative percentage of applied amount Harmine permeated (%). Mean ± SD are given.

| **Formulation** | **2 h** | **4 h** | **6 h** | **8 h** | **24 h** |
|---|---|---|---|---|---|
| ISM21007, 6% GZ17-6.02 MG cream, untreated | 0.002 ± 0.002 | 0.009 ± 0.008 | 0.022 ± 0.017 | 0.039 ± 0.027 | 0.211 ± 0.135 |
| ISM21008, 6% GZ17-6.02 MG cream, milled Isovanillin | 0.002 ± 0.003 | 0.011 ± 0.012 | 0.026 ± 0.024 | 0.046 ± 0.038 | 0.207 ± 0.118 |
| ISM21009, 6% GZ17-6.02 MG cream, milled GZ 17-6.02 | 0.000 ± 0.001 | 0.004 ± 0.004 | 0.014 ± 0.009 | 0.028 ± 0.014 | 0.183 ± 0.079 |

### Amount in tissue

The amount of Harmine accumulated in dermis and epidermis are shown in the table and figures. The variability between the replicates within each formulation are large and no differences between the formulations can be seen. As for Isovanillin, a larger amount of Harmine is found in epidermis compared to dermis. One should however keep in mind that although the membranes were washed thoroughly, it cannot be guaranteed that all remaining of the formulation is removed.

### Amount Harmine found in the tissue, Mean ± SD are given.

| **Formulation** | **Amount in Dermis (µg)** | **Amount in Dermis (µg/g)** | **Amount in Epidermis (µg)** | **Amount in Epidermis (µg/g)** |
|---|---|---|---|---|
| ISM210078, 6% GZ17-6.02 MG cream, untreated | 1.30 ± 0.88 | 38.4 ± 27.8 | 2.09 ± 0.52 | 408.5 ± 100.7 |
| ISM21008, 6% GZ17-6.02 MG cream, milled Isovanillin | 1.34 ± 0.80 | 38.9 ± 24.4 | 3.00 ± 1.17 | 585.5 ± 227.8 |
| ISM21009, 6% GZ17-6.02 MG cream, milled GZ17-6.02 | 1.77 ± 0.84 | 51.0 ± 22.8 | 3.69 ± 2.68 | 720.5 ± 522.8 |
| ISM21009, 6% GZ17-6.02 MG cream, milled GZ17-6.02, cell 11 and 12 outliers | 1.67 ± 1.13 | 51.0 ± 22.8 | 4.47 ± 3.36 | 873.7 ± 657.2 |

### Permeation of Curcumin

### Amount in receptor solution

No amount of Curcumin could be detected in the receptor solution and the amount of Curcumin in the receptor solution was hence below the detection limit (0.05 µg/ml, corresponding to a flux of 0.13 µg/cm²/h = (0.4 µg/ml*1.7 ml/h / 0.64 cm²) for all cells and in all fractions as shown in the tables.

### Cumulative amount (µg/cm²) of Curcumin permeated. Mean ± SD are given.

| **Formulation** | **2 h** | **4 h** | **6 h** | **8 h** | **24 h** |
|---|---|---|---|---|---|
| ISM21007, 6% GZ17-6.02 MG cream, untreated | n.d. | n.d. | n.d. | n.d. | n.d. |
| ISM21008, 6% GZ17-6.02 MG cream, milled Isovanillin | n.d. | n.d. | n.d. | n.d. | n.d. |
| ISM21009, 6% GZ17-6.02 MG cream, milled GZ 17-6.02 | n.d. | n.d. | n.d. | n.d. | n.d. |

### Flux (µg/cm²/h) of Curcumin permeated. Mean ± SD are given.

| **Formulation** | **2 h** | **4 h** | **6 h** | **8 h** | **24 h** |
|---|---|---|---|---|---|
| ISM21007, 6% GZ17-6.02 MG cream, untreated | n.d. | n.d. | n.d. | n.d. | n.d. |
| ISM21008, 6% GZ17-6.02 MG cream, milled Isovanillin | n.d. | n.d. | n.d. | n.d. | n.d. |
| ISM21009, 6% GZ17-6.02 MG cream, milled GZ 17-6.02 | n.d. | n.d. | n.d. | n.d. | n.d. |

### Cumulative percentage of applied amount Curcumin permeated (%). Mean ± SD are given.

| **Formulation** | **2 h** | **4 h** | **6 h** | **8 h** | **24 h** |
|---|---|---|---|---|---|
| ISM21007, 6% GZ17-6.02 MG cream, untreated | n.d. | n.d. | n.d. | n.d. | n.d. |
| ISM21008, 6% GZ17-6.02 MG cream, milled Isovanillin | n.d. | n.d. | n.d. | n.d. | n.d. |
| ISM21009, 6% GZ17-6.02 MG cream, milled *GZ17-6.02* | n.d. | n.d. | n.d. | n.d. | n.d. |

### Amount in tissue

A small amount of Curcumin was detected in the tissue extractions as shown in the table and figures. No differences between the formulations could be detected as there is a large variability between the replicates making it hard determine any differences between the formulations.

### Amount Curcumin found in the tissue, Mean ± SD are given.

| **Formulation** | **Amount in Dermis (µg)** | **Amount in Dermis (µg/g)** | **Amount in Epidermis (µg)** | **Amount in Epidermis (µg/g)** |
|---|---|---|---|---|
| ISM210078, 6% GZ17-6.02 MG cream, untreated | 0.02 ± 0.01 | 0.48 ± 0.45 | 0.02 ± 0.03 | 4.29 ± 5.20 |
| ISM21008, 6% GZ17-6.02 MG cream, milled Isovanillin | 0.01 ± 0.01 | 0.40 ± 0.23 | 0.02 ± 0.01 | 3.82 ± 1.88 |
| ISM21009, 6% GZ17-6.02 MG cream, milled GZ 17-6.02 | 0.03 ± 0.02 | 0.83 ± 0.49 | 0.03 ± 0.02 | 5.24 ± 4.38 |
| ISM21009, 6% GZ17-6.02 MG cream, milled GZ17-6.02, cell 11 and 12 outliers | 0.02 ± 0.00 | 0.50 ± 0.11 | 0.01 ± 0.00 | 2.63 ± 0.30 |

### Conclusions

An *in vitro* permeation test using pig skin membranes was performed in order to evaluate if different particle sizes of GZ17-6.02 molecules results in differences in permeation. No significant differences between the evaluated formulations could be detected regarding the amount found in the receptor or the amount found in dermis). For Isovanillin a depletion could be seen with decreased flux values after 6-8 hours while no depletion could be detected for Harmine and Curcumin.

### Cumulative amount of GZ17-6.02 substances permeated the skin into the receptor (µg/cm²)*

| **Formulation** | **Isovanillin** | **Harmine** | **Curcumin** |
|---|---|---|---|
| ISM21007, untreated | 233.8 ± 63.3 | 3.84 ± 2.36 | n.d. |
| ISM21008, milled Isovanillin | 279.9 ± 95.2 | 3.88 ± 2.14 | n.d. |
| ISM21009, milled GZ17-6.02 | 273.6 ± 72.5 | 3.43 ± 1.53 | n.d. |

| | | | |
|---|---|---|---|
| Amount in dermis (ug) divided by membrane area (cm²) | | | |

### Amount of GZ17-6.02 substances in dermis (µg/cm²)*

| **Formulation** | **Isovanillin** | **Harmine** | **Curcumin** |
|---|---|---|---|
| ISM21007, untreated | 0.72 ± 0.37 | 2.03 ± 1.37 | 0.03 ± 0.02 |
| ISM21008, milled Isovanillin | 1.56 ± 0.87 | 2.10 ± 1.25 | 0.02 ± 0.02 |
| ISM21009, milled GZ17-6.02 | 2.85 ± 2.16 | 2.77 ± 1.31 | 0.04 ± 0.02 |
| ISM21009, milled GZ17-6.02, 2 outliers | 1.29 ± 0.51 | 2.61± 1.76 | 0.03 ± 0.01 |

| | | | |
|---|---|---|---|
| *Amount in dermis (µg) divided by the membrane area (cm²) | | | |

### EXAMPLE

### IVPT experiment with finite dose 3% and 6% GZ17-6.02 MG formulation

The objective of this study was to perform an in vitro permeation test (IVPT) on pig skin for 3% and 6% GZ17-6.02 monoglyceride cream with a finite dose applied. Two formulations were evaluated in this experiment. 3% GZ17-6.02 monoglyceride cream and 6% GZ17-6.02 monoglyceride cream.

It was found that increasing concentration GZ17-6.02 in the formulation results in increased concentration of isovanillin, harmine and curcumin in both dermis and epidermis. Increased concentration in the formulation also give rise to increased permeation of isovanillin and harmine through the skin tissue. No amount of curcumin was found to permeating the skin membranes.

Correlating to previous experiment with application of infinite dose it was found that the percentage of applied amount of Isovanillin and Harmine permeating the skin membranes correlates well with the applied formulation dose. Interesting the absolute amount of Isovanillin, Harmine and Curcumin in the dermis appears to be constant and does not change when the formulation dose is altered, i.e., the percentage of applied dose found in dermis decreases with increased dose.

PBS with 0.1% Brij was used as receptor solution for all experiments. The recipe of the receptor solution is given in Table 1. Prior to the experiment the receptor solution is degassed.

**Table 1. Composition receptor solution.**

| **Chemical** | **Internal number** | **% (w/w)** | **Composition for 1000 mL** |
|---|---|---|---|
| Sodium chloride | CH0013 | 0.765 | 7.65 g |
| di-Sodium hydrogen phosphate, dihydrate | CH0012 | 0.0908 | 0.908 g |
| Potassium dihydrogen phosphate | CH0009 | 0.021 | 0.210 g |
| HPLC water | n.a | 98.00 | 980 g |
| Brij 020-SS-(RB) | CH0611 | 0.10 | 1.00 g |
| 1M NaOH or 1MHC1 | n.a | to pH 7.4 | to pH 7.4 |
| HPLC water | n.a | to volume | to 1000 mL |

### 1.1.1 Solubility of Isovanillin, harmine and curcumin

The solubility of Isovanillin, Harmine and Curcumin was determined in PBS with 0.1% Brij. Excess of GZ17-6.02 were added to the solvent, which were stirred for 1 hour and incubated at 32 °C for 24h. Thereafter the sample was centrifuged or filtrated and the solubility determined by HPLC method DM.216.03[4] or DM.217.01 [5]. One sample was prepared and duplicate injections were analysed.

### 1.2 Skin membranes

Dermatomed skin membranes from pig inner eras was used as membranes. All membranes were randomised.

### 1.3 Equipment

The Bronaugh diffusion cell equipment were used for the in vitro experiments. The equipment consists of 14 in-line cells form PermeGear with an orifice diameter of 9 mm.

### 1.4 IVPT experiment

The setup for the first experiment is given in Table 2 and application scheme is given in Table 3.

**Table 2. Experimental parameters for in vitro permeation experiment**

| **Parameter** | **Value/Interval** |
|---|---|
| Receptor phase composition | PBS with 0.1% (w/w) Brij |
| Temperature of water bath | 34 °C giving a membrane temperature of 32.0°C |
| Membrane | Full thickness skin membranes from pig ear |
| Randomisation | Membranes will be randomized |
| Application interval | One application |
| Application dose | 10 mg/cm² (6.4 mg/cell) |
| Application of formulations | 6.4 mg applied with spatula (aim to be between 6.08 mg to 6.72 mg) |
| | 1) Pre-weight spatula and beaker with formulation |
| | 2) Apply formulation on membrane |
| | 3) Weight the blunt stick to determine amount left on the stick |
| Sampling timepoint after experiment start | 1, 2, 4, 6, 8, 14, 24 h |
| Duration of study | 24 h |
| Occlusion | **No occlusion** |
| Analysis | RP-HPLC will be performed according to DM.217.01 and MS method developed at EHS analytical |
| Post experiment | Clean the membranes, separate epidermis and dermis and extract substances from the membranes. |

**Table 3. Application scheme for IVPT experiment**

| **Cell** | **Formulation** | **Batch** | **Applied amount** |
|---|---|---|---|
| 1-7 | 3% GZ17-6.02 monoglyceride cream | ISM22012 | 10 mg/cm² |
| 8-14 | 6% GZ17-6.02 monoglyceride cream | ISM22003 | 10 mg/cm² |

### 1.4.1 Extraction of substances from the skin

After the in vitro experiment is terminated each membrane were washed, dismounted and prepared according to the following procedure:
1. Wash the membrane six times with 0.5 ml PBS buffer and collect the PBS in the wash fraction tubes.
2. Dismount the cells and gently clean the membranes with PBS moisture tops. Collect the tops in the wash fraction tubes.
3. Gently separate stratum corneum from dermis by peeling the membrane and collect in a test tube.
4. Cut out the cell flange (the unexposed skin) with a 10 mm punch and collect the material in a test tube.
5. Cut the dermis into thin strips with a scalpel and collect the material in a test tube 1.5 ml.
6. Add 1.4 ml of methanol/water (90:10) to the samples.
7. Vortex the sample and sonicate for 30 minutes in cold water (2-8°C add ice to the bath).
8. The samples should then be centrifuged at 14000 rpm and transferred to HPLC vial for analysis.

### 1.4.2 Analysis

Analysis of Harmine, Curcumin and Isovanillin both in receptor samples and skin extractions were analyzed by LC-MS. Analysis was performed by reversed phase LC-MSMS on a Quattro Micro triple quadrupole mass spectrometer (Waters, Altricham, Cheshire, UK) in the electrospray-mode monitoring positive ions (ESI+). Capillary voltage was 3.0 kV and the temperature of the ion source was 130 °C, and the desolvation temperature was 250 °C. Collision-induced dissociation was performed using argon as collision gas and the pressure of the collision cell was 3 × 10-3 mbar. For quantification, multiple-reaction monitoring (MRM) was performed: Curcumin (369-177), d6-curcumin (375-180), harmine (213-198), d-harmine (216→ 198) and isovanillin (153-93). The monitored ions were individually optimized.

The MS instrument was connected to LC pumps (Shimadzu LC10ADVP, Shimadzu Inc., Kyoto, Japan). Gradient elution was performed from 85/15/0.05 to 40/60/0.05 water/acetonitrile/formic acid in 6 minutes. Partially filled loop injections of 6 µl in a 20 µl loop volume containing 14 µl of a focus liquid of 5/95 (% v/v) acetonitrile/water solution was made with a CTC-pal autosampler (CTC Analytics AG, Zwingen, Switzerland). The flow rate was 0.2 ml/min, and the LC column was an Ascentis Express C18, 5cm x 2.2 mm, 2.7µm (Supelco, PA, US). The quantification limit for Harmine and Curcumin was 1 ng/mL while the quantification limit was 25 ng/ml for Isovanillin. Analysis was also made by RP-HPLC-UV.

### 2 Results and discussion

### 2.1 Solubility of in the receptor solution

The solubility of Isovanillin, Harmine and Curcumin was determined in PBS with 0.1% Brij. The results of the solubility study are shown in Table 4. The maximum obtained concentration in the receptor solution in previous IVPT experiment was 18 µg/mL Isovanillin, 0.13 µg/ml Harmine and 0 µg/mL Curcumin. It was hence judged the solubility was more than 10 times higher than the expected concentration and sink condition should be maintained during the experiment.

**Table 4. Solubility in receptor solution (mg/mL)**

| **Solvent** | **Solubility Isovanillin mg/mL** | **Solubility Harmine mg/mL** | **Solubility Curcumin mg/mL** |
|---|---|---|---|
| PBS buffer with 0.1% Brij | 3.96 ± 0.03 | 0.05 ± 0.00 | 0.01± 0.00 |

### 2.2 Comparison between LC-MSMS and HPLC-UV analysis

There was a good agreement with the results between the LC-MSMS analysis and HPLC-UV analysis. However, for most samples the concentration was below the quantification limit for the HPLC-UV method. As similar results were obtained for the two analysis methods but only one full set of data was obtained with LC-MSMS the concentrations obtained in the LC-MSMS analysis were used to calculate the results.

### 2.3 Permeation through the skin tissues

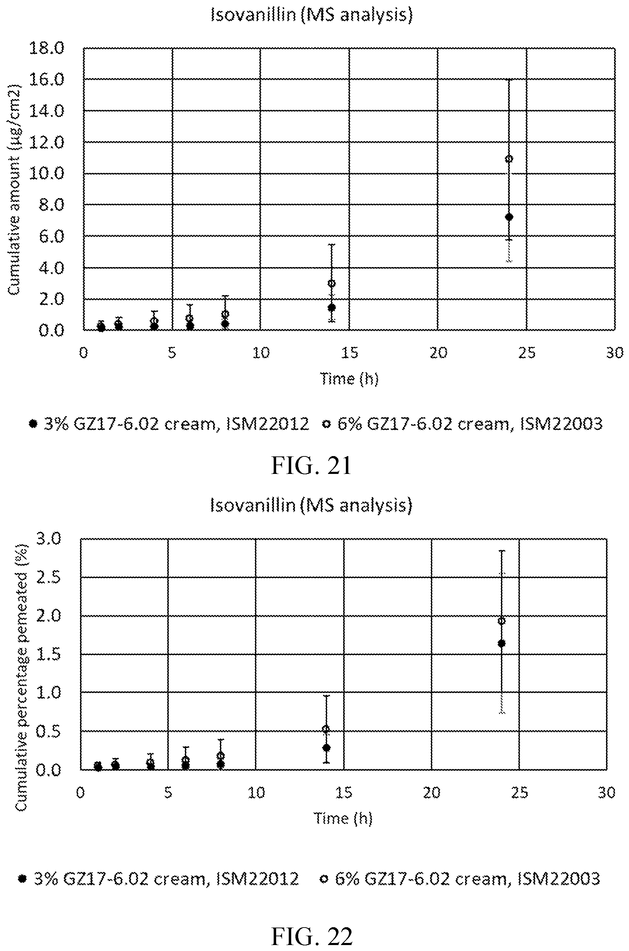

Isovanillin was found to permeate the skin tissue although only about 2% of the applied dose had permeated the skin after 24 h, see Fig. 21 and Fig. 22. A small permeation of Harmine could also be seen. However, the permeation was just in the border of the quantification limit (QL) and for many of the replicates the amount permeated was below the quantification limit. Hence, no graph is presented for Harmine as it would be misleading considering the fact that most of the replicates had concentrations below the quantification limit. The quantification limit for Harmine was 1 ng/ml and corresponding to 2.7 ng/cm²/h (1 ng/mL * 1.7 mL/h / 0.64 cm²). No permeation of curcumin could be detected in any of the receptor solution samples. The quantification limit was 1 ng/mL for Curcumin and hence less than 2.7 ng/cm²/h Curcumin (1 ng/mL *1.7 mL/h / 0.64 cm²) permeates the skin membranes.

For two of the replicates (cell 7 and cell 13) a significantly large amount of both Isovanillin and Harmine were found to permeate in the first fractions, in contradiction to all other replicates, see Table 5, Table 6, Table 7 and Table 8, where European style decimal commas are used in reporting the values. It was hence clear that these membranes could be considered as outliers and they were not used in any of the results calculations.

**Table 5. Cumulative Isovanillin permeated for 3% GZ17-6.02 monoglyceride cream, µg/cm²**

| **Replicate** | **1 h** | **2 h** | **4 h** | **6 h** | **8 h** | **14 h** | **24 h** |
|---|---|---|---|---|---|---|---|
| Cell 1 | 0,083 | 0,174 | 0,174 | 0,174 | 0,174 | 0,606 | 3,345 |
| Cell 2 | 0,108 | 0,189 | 0,189 | 0,189 | 0,189 | 1,007 | 7,124 |
| Cell 3 | 0,357 | 0,469 | 0,469 | 0,645 | 0,909 | 2,720 | 10,131 |
| Cell 4 | 0,148 | 0,272 | 0,272 | 0,272 | 0,272 | 1,639 | 9,875 |
| Cell 5 | 0,276 | 0,370 | 0,370 | 0,513 | 0,685 | 1,910 | 8,259 |
| Cell 6 | 0,000 | 0,074 | 0,074 | 0,074 | 0,240 | 0,884 | 4,466 |
| Cell 7* | 4,626 | 6,035 | 6,885 | 7,359 | 7,795 | 9,868 | 16,514 |
| **Mean** | **0,16** | **0,26** | **0,26** | **0,31** | **0,41** | **1,46** | **7,20** |
| **SD** | **0,13** | **0,14** | **0,14** | **0,22** | **0,31** | **0,79** | **2,80** |
| **RSD** | **81,15** | **55,56** | **55,56** | **70,90** | **75,04** | **53,80** | **38,89** |

| | | | | | | | |
|---|---|---|---|---|---|---|---|
| *Outlier not used in any of the calculations | | | | | | | |

**Table 6. Cumulative Isovanillin permeated for 6% GZ17-6.02 monoglyceride cream, µg/cm²**

| **Replicate** | **1 h** | **2 h** | **4 h** | **6 h** | **8 h** | **14 h** | **24 h** |
|---|---|---|---|---|---|---|---|
| Cell 8 | 0,086 | 0,086 | 0,086 | 0,086 | 0,242 | 1,276 | 8,780 |
| Cell 9 | 0,000 | 0,000 | 0,000 | 0,000 | 0,000 | 0,681 | 6,491 |
| Cell 10 | 0,364 | 0,496 | 0,681 | 0,928 | 1,342 | 4,537 | 14,906 |
| Cell 11 | 0,388 | 0,532 | 0,831 | 1,103 | 1,519 | 4,512 | 12,826 |
| Cell 12 | 0,803 | 1,162 | 1,683 | 2,291 | 3,066 | 6,387 | 17,733 |
| Cell 13* | 4,501 | 7,383 | 9,799 | 11,555 | 13,037 | 18,966 | 34,062 |
| Cell 14 | 0,073 | 0,073 | 0,073 | 0,073 | 0,073 | 0,680 | 4,612 |
| **Mean** | **0,29** | **0,39** | **0,56** | **0,75** | **1,04** | **3,01** | **10,89** |
| **SD** | **0,30** | **0,44** | **0,65** | **0,89** | **1,19** | **2,44** | **5,10** |
| **RSD** | **105,07** | **112,63** | **116,58** | **119,66** | **114,38** | **81,13** | **46,80** |

| | | | | | | | |
|---|---|---|---|---|---|---|---|
| *Outlier not used in any of the calculations | | | | | | | |

**Table 7. Cumulative Harmine permeated for 3% GZ17-6.02 monoglyceride cream, µg/cm²**

| **Replicate** | **1 h** | **2 h** | **4 h** | **6 h** | **8 h** | **14 h** | **24 h** |
|---|---|---|---|---|---|---|---|
| Cell 1 | <QL | <QL | <QL | <QL | <QL | <QL | <QL |
| Cell 2 | <QL | <QL | <QL | <QL | <QL | <QL | 0,015 |
| Cell 3 | <QL | <QL | <QL | 0,005 | 0,012 | 0,047 | 0,154 |
| Cell 4 | <QL | <QL | <QL | <QL | <QL | <QL | <QL |
| Cell 5 | <QL | <QL | <QL | <QL | <QL | 0,009 | 0,051 |
| Cell 6 | <QL | <QL | <QL | <QL | <QL | <QL | <QL |
| Cell 7* | 0,011 | 0,062 | 0,208 | 0,397 | 0,607 | 1,317 | 2,393 |
| **Mean** | **0,00** | **0,00** | **0,00** | **0,00** | **0,00** | **0,01** | **0,04** |
| **SD** | **0,00** | **0,00** | **0,00** | **0,00** | **0,00** | **0,02** | **0,06** |
| **RSD** | **-** | **-** | **-** | **244,95** | **244,95** | **200,80** | **165,72** |

| | | | | | | | |
|---|---|---|---|---|---|---|---|
| *Outlier not used in any of the calculations | | | | | | | |

**Table 8. Cumulative Harmine permeated for 6% GZ17-6.02 monoglyceride cream, µg/cm²**

| **Replicate** | **1 h** | **2 h** | **4 h** | **6 h** | **8 h** | **14 h** | **24 h** |
|---|---|---|---|---|---|---|---|
| Cell 8 | <QL | <QL | 0,005 | 0,011 | 0,019 | 0,047 | 0,115 |
| Cell 9 | <QL | <QL | <QL | <QL | <QL | 0,012 | 0,050 |
| Cell 10 | <QL | <QL | 0,005 | 0,012 | 0,021 | 0,076 | 0,258 |
| Cell 11 | <QL | <QL | 0,007 | 0,021 | 0,043 | 0,176 | 0,536 |
| Cell 12 | 0,003 | 0,012 | 0,043 | 0,076 | 0,112 | 0,211 | 0,405 |
| Cell 13* | 0,008 | 0,065 | 0,238 | 0,439 | 0,645 | 1,353 | 2,574 |
| Cell 14 | <QL | 0,003 | 0,008 | 0,013 | 0,013 | 0,013 | 0,034 |
| **Mean** | **0,00** | **0,00** | **0,01** | **0,02** | **0,03** | **0,09** | **0,23** |
| **SD** | **0,00** | **0,00** | **0,02** | **0,03** | **0,04** | **0,09** | **0,20** |
| **RSD** | **-** | **-** | **137,8** | **122,5** | **116,5** | **95,4** | **87,6** |

| | | | | | | | |
|---|---|---|---|---|---|---|---|
| *Outlier not used in any of the calculations | | | | | | | |

### 2.4 Amount in epidermis

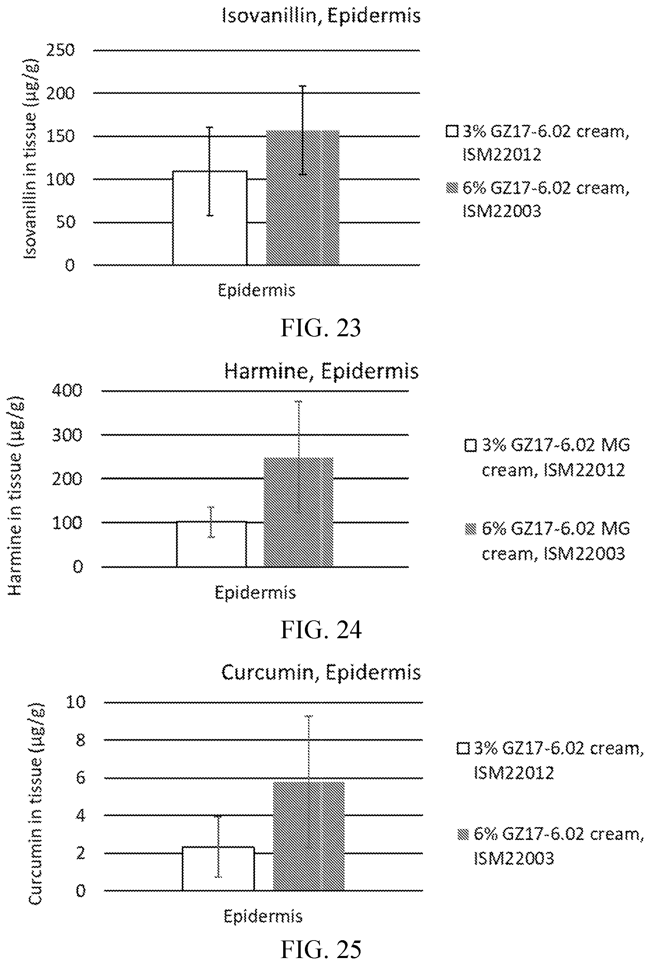

All three GZ17-6.02 substances were found in epidermis after application of both 3% and 6% GZ 17-6.02 monoglyceride crem, see Fig. 23, Fig. 24, Fig. 25, Table 9 and Table 10. A higher concentration of Isovanillin, Harmine and Curcumin were found in epidermis after application of formulation containing 6% GZ17-6.02 compared to 3% GZ17-6.02.

**Table 9. Amount of GZ17-6.02 in epidermis after application of 6% and 3% GZ17-6.02 monoglyceride cream**

| **Formulation** | **Isovanillin µg/g** | **Harmine µg/g** | **Curcumin µg/g** |
|---|---|---|---|
| **3% MG cream** | 109.3 ± 51.6 | 102.1 ± 33.7 | 2.33 ± 1.61 |
| **6% MG cream** | 157.2 ± 110.0 | 249.0 ± 126.9 | 5.79 ± 3.49 |

**Table 10. Percentage of applied amount GZ17-6.02 found in epidermis after application of 6% and 3% GZ17-6.02 monoglyceride cream**

| **Formulation** | **Isovanillin %** | **Harmine %** | **Curcumin %** |
|---|---|---|---|
| **3% MG cream** | 0.16 ± 0.08 | 0.92 ± 0.30 | 0.03 ± 0.02 |
| **6% MG cream** | 0.23 ± 0.18 | 2.15 ± 1.30 | 0.07 ± 0.04 |

### 2.5 Amount in dermis

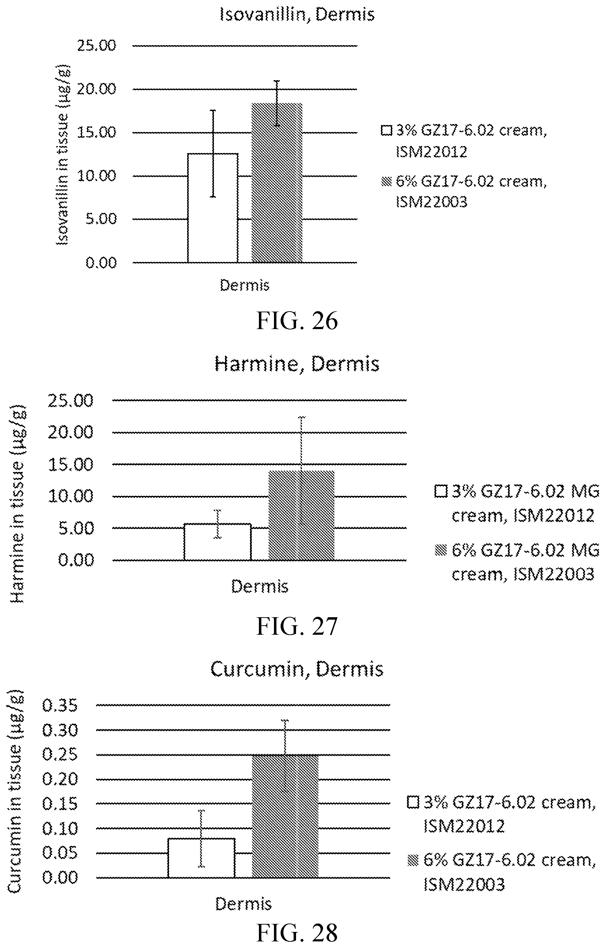

All three GZ17-6.02 substances were found in dermis after application of both 3% and 6% GZ17-6.02 monoglyceride crem, see Fig. 26, Fig. 27, Fig. 28, Table 11, and Table 12. A higher concentration of Isovanillin, Harmine and Curcumin were found in dermis after application of formulation containing 6% GZ17-6.02 compared to 3% GZ17-6.02.

**Table 11. Amount of GZ17-6.02 in dermis after application of 6% and 3% GZ17-6.02 monoglyceride cream**

| **Formulation** | **Isovanillin µg/g** | **Harmine µg/g** | **Curcumin µg/g** |
|---|---|---|---|
| **3% MG cream** | 12.6 ± 5.0 | 5.7 ± 2.2 | 0.08 ± 0.06 |
| **6% MG cream** | 18.4 ± 2.6 | 14.1 ± 8.4 | 0.25 ± 0.07 |

**Table 12. Percentage of applied amount GZ17-6.02 found in dermis after application of 6% and 3% GZ17-6.02 monoglyceride cream**

| **Formulation** | **Isovanillin %** | **Harmine %** | **Curcumin %** |
|---|---|---|---|
| **3% MG cream** | 0.15 ± 0.06 | 0.39 ± 0.15 | 0.01 ± 0.00 |
| **6% MG cream** | 0.20 ± 0.04 | 0.86 ± 0.41 | 0.02 ± 0.01 |

### 2.6 Comparison to previous studies

In this study we applied a finite dose (10 mg/cm²) in order to use the same dose as planned in upcoming clinical study. Previously we have applied a much larger dose (230 mg/cm²) in order ensure that we can detect the substances.

### 2.6.1 Correlation of amount permeated

The cumulative amount of Isovanillin, Harmine and Curcumin which has permeated the tissue in this and previous experiment is shown in Table 13 and Table 14. As can be seen the cumulative amount permeated (µg/g) increases significantly when the dose of applied formulation increases. The percentage of applied dose which permeates (%) the tissue appears to be the same.

**Table 13. Cumulative GZ17-6.02 (µg/cm²) permeating the skin membranes after 24 h application of finite (10 mg/cm²) or infinite dose (230 mg/cm²)**

| **Formulation dose mg/cm²** | **Formulation** | **Isovanillin µg/cm²** | **Harmine µg/cm²** | **Curcumin µg/cm²** |
|---|---|---|---|---|
| 10 | 3% GZ17-6.02 cream | 7.2 ± 2.8 | 0.04 ± 0.06 | n.d. |
| 10 | 6% GZ17-6.02 cream | 10.9 ± 5.1 | 0.23 ± 0.20 | n.d. |
| 230 | 6% MG cream, milled Isovanillin | 233.8 ± 63.3 | 3.84 ± 2.36 | n.d. |
| 230 | 6% MG cream, milled GZ17-6.02 | 279.9 ± 95.2 | 3.88 ± 2.14 | n.d. |
| 230 | 6% MG cream, untreated GZ17-6.02 | 272.6 ± 92.8 | 3.61 ± 2.04 | n.d. |

**Table 14. Percentage of applied dose GZ17-6.02 (%) permeating the skin membranes after 24 h application of finite (10 mg/cm²) or infinite dose (230 mg/cm²)**

| **Formulation dose mg/cm²** | **Formulation** | **Isovanillin µg/cm²** | **Harmine µg/cm²** | **Curcumin µg/cm²** |
|---|---|---|---|---|
| 10 | 3% GZ17-6.02 cream | 1.6 ± 0.9 | 0.05 ± 0.08 | n.d. |
| 10 | 6% GZ17-6.02 cream | 1.0 ± 0.9 | 0.23 ± 0.19 | n.d. |
| 230 | 6% MG cream, milled Isovanillin | 2.1 ± 0.6 | 0.21 ± 0.14 | n.d. |
| 230 | 6% MG cream, milled GZ17-6.02 | 2.5 ± 0.9 | 0.21 ± 0.12 | n.d. |
| 230 | 6% MG cream, untreated GZ17-6.02 | 2.4 ± 0.8 | 0.19 ± 0.11 | n.d. |

### 2.6.2 Correlation of amount in dermis

The amount of Isovanillin, Harmine and Curcumin accumulated in dermis appears to be unaffected by the applied dose and approximately same amount of the substances are found in dermis after application of 10 mg/cm² or 230 mg/cm². Small differences between the absolute amount exists which could due to the fact that skin samples from different pig individuals have been used in the two separate experiments. Nevertheless, the differences do not correlate with the very large differences between the applied amount formulation.

**Table 15. Amount of GZ17-6.02 (µg/g) in dermis after application of finite (10 mg/cm²) or infinite dose (230 mg/cm²)**

| **Formulation dose mg/cm²** | **Formulation** | **Isovanillin µg/cm²** | **Harmine µg/cm²** | **Curcumin µg/cm²** |
|---|---|---|---|---|
| 10 | 3% GZ17-6.02 cream | 12.6 ± 5.0 | 5.7 ± 2.2 | 0.08 ± 0.06 |
| 10 | 6% GZ17-6.02 cream | 18.4 ± 2.6 | 14.1 ± 8.4 | 0.25 ± 0.07 |
| 230 | 6% MG cream, milled Isovanillin | 13.4 ± 6.3 | 38.4 ± 27.8 | 0.48 ± 0.14 |
| 230 | 6% MG cream, milled GZ17-6.02 | 29.4 ± 19.3 | 38.9 ± 24.4 | 0.40 ± 0.23 |
| 230 | 6% MG cream, untreated GZ17-6.02 | 23.6 ± 8.6 | 47.5 ± 30.8 | 0.50 ± 0.11 |

**Table 16. Percentage of applied dose GZ17-6.02 (%) in dermis after application of finite (10 mg/cm²) or infinite dose (230 mg/cm²)**

| **Formulation dose mg/cm²** | **Formulation** | **Isovanillin %** | **Harmine %** | **Curcumin %** |
|---|---|---|---|---|
| 10 | 3% GZ17-6.02 cream | 0.15 ± 0.06 | 0.39 ± 0.15 | 0.01 ± 0.00 |
| 10 | 6% GZ17-6.02 cream | 0.20 ± 0.04 | 0.86 ± 0.41 | 0.02 ± 0.01 |
| 230 | 6% MG cream, milled Isovanillin | 0.01 ± 0.00 | 0. 11 ± 0.07 | 0.00 ± 0.00 |
| 230 | 6% MG cream, milled GZ17-6.02 | 0.01 ± 0.01 | 0.11 ± 0.06 | 0.00 ± 0.00 |
| 230 | 6% MG cream, untreated GZ17-6.02 | 0.01 ± 0.00 | 0. 14 ± 0.09 | 0.00 ± 0.00 |

### 3 Conclusions

An in vitro permeation test (IVPT) was performed in order to investigate the difference between application of a 3% GZ17-6.02 monoglyceride cream and a 6% GZ17-6.02 monoglyceride cream. A finite dose was applied giving a correlation to the *in-vivo* situation. It was found that increasing concentration GZ 17-6.02 in the formulation results in increased concentration of Isovanillin, Harmine and curcumin in both dermis and epidermis. Increased concentration in the formulation also give rise to increased permeation of Isovanillin and Harmine through the skin tissue. No amount of curcumin was found to permeating the skin membranes.

Correlating to previous experiment with application of infinite dose it was found that the percentage of applied amount of Isovanillin and Harmine permeating the skin membranes correlates well with the applied formulation dose. Interestingly, the absolute amount of Isovanillin, Harmine and Curcumin in the dermis appears to be constant and does not change when the formulation dose is altered, i.e. the percentage of applied dose found in dermis decreases with increased dose.

### EXAMPLE

Actinic keratoses (AK) are premalignant intraepidermal lesions with limited treatment options. Here we investigate the efficacy of GZ21T, a novel anti-tumor monoglyceride formulation composed of curcumin, harmine, and isovanillin, in the treatment of AK.

An oral formulation (GZ17-6.02) and topical preparation (GZ21T) comprised of the same fixed, stoichiometric ratio of curcumin (10%), harmine (13%), and isovanillin (77%) have been developed. Together, the three active ingredients killed AK cells as more effectively than any of its component parts as either individual agents or when combined in pairs. The three active ingredients caused greater levels of DNA damage than any of its component parts as either individual agents or when combined in pairs. As a single agent, compared to isolated components, GZ17-6.02/GZ21T caused significantly greater activation of PKR-like endoplasmic reticulum kinase, the AMP-dependent protein kinase and ULK1 and significantly reduced the activities of mTORC1, AKT and YAP. Knock down of the autophagy-regulatory proteins ULK1, Beclin1 or ATG5 significantly reduced the lethality of GZ17-6.02/GZ21T alone. Expression of an activated mTOR mutant suppressed autophagosome formation and autophagic flux, and reduced tumor cell killing. Blockade of both autophagy and death receptor signaling abolished drug-induced AK cell death.

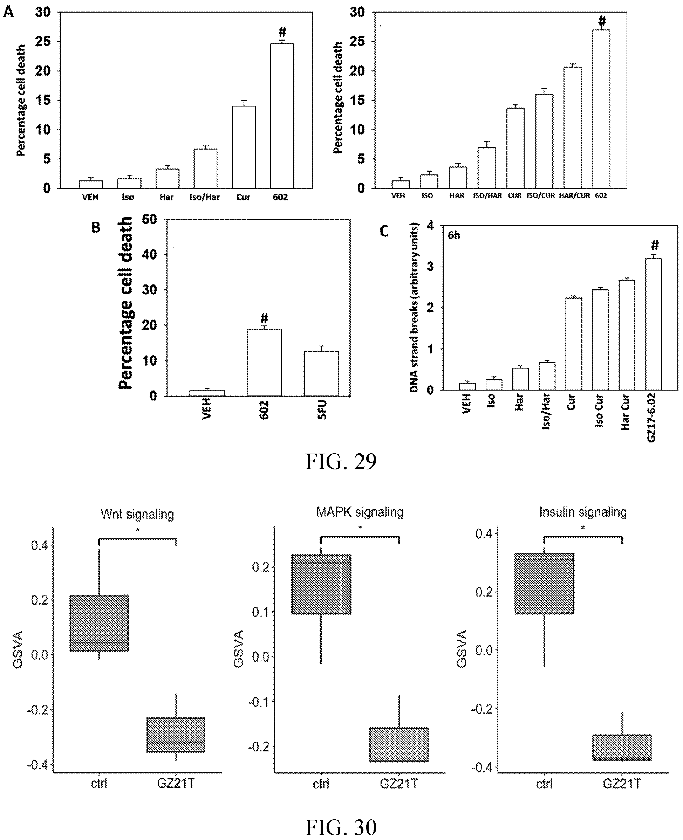

This assay revealed a significantly greater percentage of cell death in cells treated with GZ17-6.02/GZ21T compared to curcumin, harmine, and isovanillin, both alone and in pairs (Fig. 29A). Finally, a comet assay was performed to assess GZ17-6.02/GZ21T's ability to induce DNA breaks and crosslinks in AK cells. This analysis revealed that GZ17-6.02/GZ21T was more effective at causing DNA damage than its individual components, as single agents or in pairs (Fig. 29C).

Materials and Methods: A Cell Titer Glo assay was used to determine the sensitivity of EGF-stimulated HaCaT cells to GZ21T. Annexin-V assay was utilized to assess the pro-apoptotic effects of GZ21T on HaCaT cells, and the mechanism was clarified by western blot. AK lesions were induced in Female SKH-1 mice by exposing them to UVB radiation (500 J/m2) five times weekly for ten weeks. Mice were subsequently treated with 0.25 grams topical GZ21T or control cream five days per week. At the studies endpoint, lesional samples were collected from control and GZ21T treated mice and were subjected to RNA-sequencing and reverse phase protein arrays (RPPA). Gene set enrichment analysis (GSEA) was performed using the KEGG database as reference to identify the top pathways altered by GZ21T treatment. Gene set variation analysis (GSVA) was used to assess GZ21T's effect on pathways previously implicated in the pathogenesis of AK. Enrichment analysis was also performed on proteins showing decreased expression following GZ21T treatment using Enrichr with the KEGG database as reference.

In a pilot test, a formulation was prepared using the triple active agents with monoglycerides, citric acid pH-modifier, and water.

| Ingredients | Quantity % (w/w) |
|---|---|
| GZ17-6.02: | 6.0% consisting of: |
| Isovanillin | 4.62 |
| Harmine | 0.78 |
| Curcumin | 0.60 |
| Glyceryl monomyristate | 18.0 |
| Glyceryl monolaurate | 6.0 |
| Citric acid, anhydrous | 0.5 |
| Water, purified | 69.5 |
| Total | 100 |

In the final study, the following formulation was used to prepare GZ21T topical formulation:

| **Ingredients** | **Quantity % (w/w)** |
|---|---|
| GZ17-6.02: | 6.0% consisting of: |
| Isovanillin, micronized | 4.62 |
| Harmine | 0.78 |
| Curcumin | 0.60 |
| Glyceryl monomyristate | 18.0 |
| Glyceryl monolaurate | 6.0 |
| Polyoxyethylene (100) stearate | 1.0 |
| Glycerol | 5.0 |
| Citric acid, anhydrous | 0.5 |
| Water, purified | 63.5 |
| **Total** | **100** |

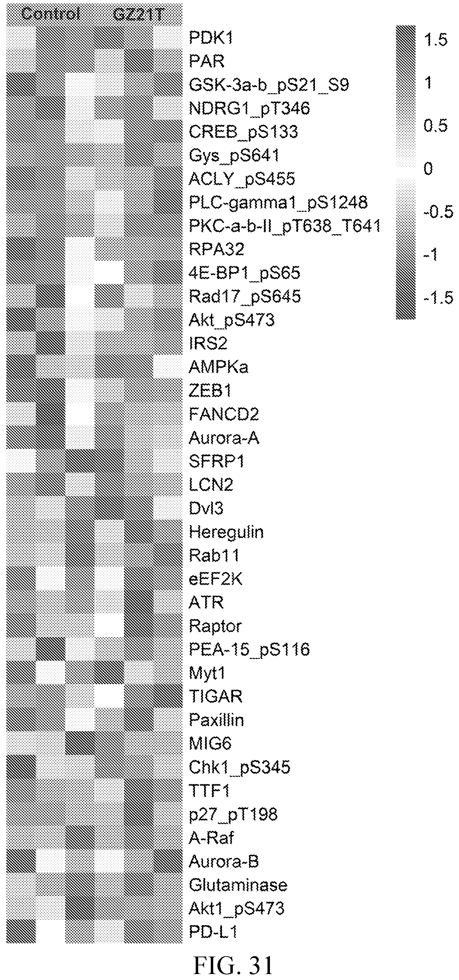

Results: GZ21T inhibited the growth of EGF-simulated HaCaT cells with an IC50±SEM value of 5.07±0.78 µg/ml. As GZ21T concentration increased, an increase in the percentage of HaCaT cells in early (p=0.041) and late apoptosis (p=0.029) was observed, which was partially mediated by PARP cleavage. After 40 days of treatment, mice receiving GZ21T demonstrated a significant decrease in lesion count (p=0.028) and surface area occupied by tumor (p=0.026). Gene Set Enrichment Analysis (GSEA) showed that GZ21T downregulates pathways related to cell cycle progression and metabolism such as DNA replication, cell cycle, and oxidative phosphorylation in AK lesions. Gene Set Variation Analysis (GSVA) revealed (Fig. 30) downregulation of MAPK signaling (p=0.026), Wnt signaling (p=0.044), and insulin signaling (p=0.021). RPPA showed that GZ21T treatment suppresses key proteins such as Raf (p=0.003), glutaminase (p=0.004), and PDL1 (p=0.033). Enrichment analysis of proteomics data demonstrated downregulation of central carbon metabolism, ErbB signaling, cell cycle, and insulin signaling in GZ21T treated mice (Fig. 31).

Conclusion: GZ21T inhibits the growth and progression of AK through suppression of targets related to cell cycle progression, MAPK signaling, Wnt signaling, and insulin signaling and holds promise as a novel therapeutic option for the treatment of AKs.

## Claims

1. A topical formulation for enhanced stability and delivery of active agents, said formulation comprising a dispersion of crystalline monoglycerides in an aqueous carrier, and at least two active agents selected from the group consisting of an isovanillin component, a harmine component, and a curcumin component, wherein said at least two active agents are stabilized in said dispersion of crystalline monoglycerides, wherein said monoglycerides are selected from glycerol monolaurate, glycerol monomyristate, and mixtures thereof.

2. The topical formulation of claim 1, consisting essentially of said at least two active agents, preferably three of said active agents, and said dispersion of crystalline monoglycerides.

3. The topical formulation of any one of the foregoing claims, wherein said dispersion of crystalline monoglycerides forms a self-supporting matrix in which said at least two active agents are distributed.

4. The topical formulation of any one of the foregoing claims, wherein said monoglyceride crystals have a melting point of 32 to 38°C.

5. The topical formulation of claim 1, wherein said glycerol monolaurate and glycerol monomyristate are in a weight ratio of 1:10 to 10:1

6. The topical formulation of any one of the foregoing claims, comprising from about 3 to about 35% by weight of said crystalline monoglycerides.

7. The topical formulation of any one of the foregoing claims, where the formulation is the form of a cream, a solution, a suspension, an emulsion, a spray, a lotion, a foam, a stick, a salve, or an ointment.

8. The topical formulation of any one of the foregoing claims, further comprising one or more components selected from the group consisting of buffering agents, acids, surfactants, thickening agents, humectants, and emollients.

9. The topical formulation of any one of the foregoing claims, further comprising one or more components selected from the group consisting of celluloses, polysorbates, polyoxyethylene stearates, citric acid, lactic acid, glycerol, propylene glycol, and hyaluronic acid.

10. The topical formulation of any one of the foregoing claims, said curcumin being present at a level of from about 5-40% by weight, said harmine being present at a level of from about 7-50% by weight, and said isovanillin being present at a level of from about 25-85% by weight, based upon the total weight of the active agents, taken as 100% by weight.

11. The topical formulation of any one of the foregoing claims, each of said curcumin, harmine, and isovanillin being respectively selected from the group consisting of the esters, metal complexes, and pharmaceutically acceptable salts of the curcumin, harmine, and isovanillin, and mixtures thereof.

12. The topical formulation of any one of the foregoing claims, said curcumin being 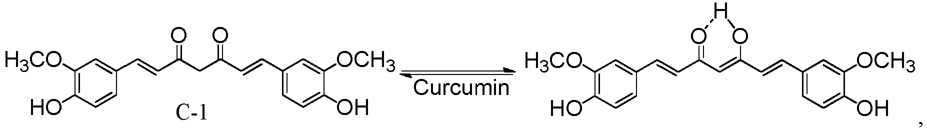
said harmine being 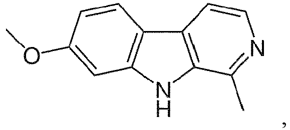 and
said isovanillin being

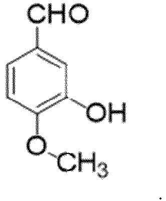

13. The topical formulation of any one of the foregoing claims, wherein said active agents maintain therapeutically-effective activity levels after 6 months in storage under ambient conditions.

14. A topical formulation of any one of the foregoing claims for use in treating a skin, dermatological, or epithelial-related condition, comprising topically applying said topical formulation to a surface of a body part of a subject in need thereof.

15. The topical formulation for use according to claim 14, wherein said topical formulation is applied in a desired thickness to said body part, wherein said active agents penetrate the skin of said body part and have a synergistic therapeutic effect in treating said skin, dermatological, or epithelial-related condition, and preferably
further comprising reapplying said topical formulation hourly, daily, weekly, bi-weekly, monthly, or as-needed.

## Patentansprüche

1. Topische Formulierung zur Verbesserung der Stabilität und Freisetzung von Wirkstoffen, wobei die Formulierung eine Dispersion von kristallinen Monoglyceriden in einem wässrigen Träger und mindestens zwei Wirkstoffe umfasst, die aus der Gruppe bestehend aus einer Isovanillin-Komponente, einer Harmin-Komponente und einer Curcumin-Komponente ausgewählt sind, wobei die mindestens zwei Wirkstoffe in der Dispersion von kristallinen Monoglyceriden stabilisiert sind, wobei die Monoglyceride aus Glycerinmonolaurat, Glycerinmonomyristat und Mischungen davon ausgewählt sind.

2. Topische Formulierung nach Anspruch 1, die im Wesentlichen aus den mindestens zwei Wirkstoffen, vorzugsweise drei der Wirkstoffe, und der Dispersion kristalliner Monoglyceride besteht.

3. Topische Formulierung nach einem der vorhergehenden Ansprüche, wobei die Dispersion kristalliner Monoglyceride eine sich selbst tragende Matrix bildet, in der die mindestens zwei Wirkstoffe verteilt sind.

4. Topische Formulierung nach einem der vorhergehenden Ansprüche, wobei die Monoglyceridkristalle einen Schmelzpunkt von 32 bis 38 °C aufweisen.

5. Topische Formulierung nach Anspruch 1, wobei das Glycerinmonolaurat und das Glycerinmonomyristat in einem Gewichtsverhältnis von 1:10 bis 10:1 vorliegen.

6. Topische Formulierung nach einem der vorhergehenden Ansprüche, die etwa 3 bis etwa 35 Gew.-% der kristallinen Monoglyceride umfasst.

7. Topische Formulierung nach einem der vorhergehenden Ansprüche, wobei die Formulierung in Form einer Creme, einer Lösung, einer Suspension, einer Emulsion, eines Sprays, einer Lotion, eines Schaums, eines Stifts, einer Salbe oder eines Balsams vorliegt.

8. Topische Formulierung nach einem der vorhergehenden Ansprüche, die weiterhin eine oder mehrere Bestandteile umfasst, die aus der Gruppe bestehend aus Puffermitteln, Säuren, Tensiden, Verdickungsmitteln, Feuchthaltemitteln und Weichmachern ausgewählt sind.

9. Topische Formulierung nach einem der vorhergehenden Ansprüche, die weiterhin eine oder mehrere Bestandteile umfasst, die aus der Gruppe bestehend aus Cellulose, Polysorbaten, Polyoxyethylenstearaten, Zitronensäure, Milchsäure, Glycerin, Propylenglykol und Hyaluronsäure ausgewählt sind.

10. Topische Formulierung nach einem der vorhergehenden Ansprüche, wobei das Curcumin in einer Menge von etwa 5 bis 40 Gew.-%, das Harmin in einer Menge von etwa 7 bis 50 Gew.-% und das Isovanillin in einer Menge von etwa 25 bis 85 Gew.-%, bezogen auf das Gesamtgewicht der Wirkstoffe, das als 100 Gew.-% angenommen wird, enthalten sind.

11. Topische Formulierung nach einem der vorhergehenden Ansprüche, wobei Curcumin, Harmin und Isovanillin jeweils aus der Gruppe bestehend aus Estern, Metallkomplexen und pharmazeutisch verträglichen Salzen von Curcumin, Harmin und Isovanillin sowie Mischungen davon ausgewählt sind.

12. Topische Formulierung nach einem der vorhergehenden Ansprüche, wobei das Curcumin 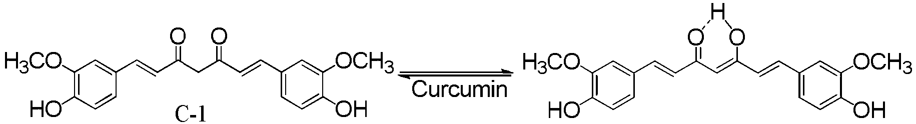 ist,
das Harmin 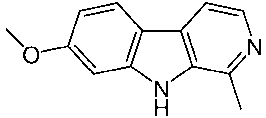 ist, und
das Isovanillin 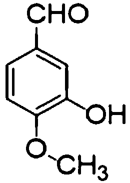 ist.

13. Topische Formulierung nach einem der vorhergehenden Ansprüche, wobei die Wirkstoffe nach 6 Monaten Lagerung unter Umgebungsbedingungen ihre therapeutisch wirksamen Aktivitätsniveaus beibehalten.

14. Topische Formulierung nach einem der vorhergehenden Ansprüche zur Verwendung bei der Behandlung eines Haut-, dermatologischen oder epithelialbezogenen Zustands, die das topische Auftragen der topischen Formulierung auf eine Oberfläche eines Körperteils eines zu behandelnden Patienten umfasst.

15. Topische Formulierung zur Verwendung nach Anspruch 14, wobei die topische Formulierung in einer gewünschten Dicke auf den Körperteil aufgetragen wird, wobei die Wirkstoffe in die Haut des Körperteils eindringen und eine synergistische therapeutische Wirkung bei der Behandlung des Haut-, dermatologischen oder epithelialbezogenen Zustands haben, und wobei vorzugsweise ferner
die topische Formulierung stündlich, täglich, wöchentlich, zweiwöchentlich, monatlich oder bedarfsgerecht erneut aufgetragen wird.

## Revendications

1. Formulation topique pour une stabilité et une administration améliorées d'agents actifs, ladite formulation comprenant une dispersion de monoglycérides cristallins dans un support aqueux, et au moins deux agents actifs choisis dans le groupe comprenant un composant d'isovanilline, un composant d'harmine et un composant de curcumine, dans laquelle lesdits au moins deux agents actifs sont stabilisés dans ladite dispersion de monoglycérides cristallins, dans laquelle lesdits monoglycérides sont choisis parmi le monolaurate de glycérol, le monomyristate de glycérol et des mélanges de ceux-ci.

2. Formulation topique selon la revendication 1, consistant essentiellement en lesdits au moins deux agents actifs, de préférence trois desdits agents actifs, et ladite dispersion de monoglycérides cristallins.

3. Formulation topique selon l'une quelconque des revendications précédentes, dans laquelle ladite dispersion de monoglycérides cristallins forme une matrice autoportante dans laquelle lesdits au moins deux agents actifs sont distribués.

4. Formulation topique selon l'une quelconque des revendications précédentes, dans laquelle lesdits cristaux de monoglycéride présentent un point de fusion de 32 à 38°C.

5. Formulation topique selon la revendication 1, dans laquelle ledit monolaurate de glycérol et ledit monomyristate de glycérol sont à un rapport pondéral de 1:10 à 10:1

6. Formulation topique selon l'une quelconque des revendications précédentes, comprenant d'environ 3 à environ 35 % en poids desdits monoglycérides cristallins.

7. Formulation topique selon l'une quelconque des revendications précédentes, dans laquelle la formulation est sous la forme d'une crème, d'une solution, d'une suspension, d'une émulsion, d'un spray, d'une lotion, d'une mousse, d'un bâton, d'un baume ou d'une pommade.

8. Formulation topique selon l'une quelconque des revendications précédentes, comprenant en outre un ou plusieurs composants choisis dans le groupe constitué d'agents tampons, d'acides, de tensioactifs, d'agents épaississants, d'humectants et d'émollients.

9. Formulation topique selon l'une quelconque des revendications précédentes, comprenant en outre un ou plusieurs composants choisis dans le groupe constitué de celluloses, de polysorbates, de stéarates de polyoxyéthylène, d'acide citrique, d'acide lactique, de glycérol, de propylène glycol et d'acide hyaluronique.

10. Formulation topique selon l'une quelconque des revendications précédentes, ladite curcumine étant présente à un niveau d'environ 5 à 40 % en poids, ladite harmine étant présente à un niveau d'environ 7 à 50 % en poids, et ladite isovanilline étant présente à un niveau d'environ 25 à 85 % en poids, sur la base du poids total des agents actifs, pris comme 100 % en poids.

11. Formulation topique selon l'une quelconque des revendications précédentes, chacune desdites curcumine, harmine et isovanilline étant choisie respectivement dans le groupe constitué des esters, des complexes métalliques et des sels pharmaceutiquement acceptables de la curcumine, de l'harmine et de l'isovanilline, et de mélanges de celles-ci.

12. Formulation topique selon l'une quelconque des revendications précédentes, ladite curcumine étant 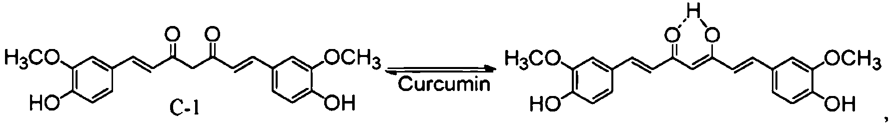 ladite harmine étant 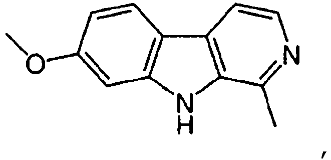 et ladite isovanilline étant

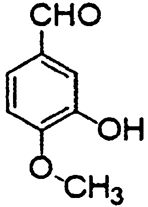

13. Formulation topique selon l'une quelconque des revendications précédentes, dans laquelle lesdits agents actifs maintiennent des niveaux d'activité thérapeutiquement efficaces après 6 mois de stockage dans des conditions ambiantes.

14. Formulation topique selon l'une quelconque des revendications précédentes pour utilisation dans le traitement d'un état cutané, dermatologique ou épithélial, comprenant l'application topique de ladite formulation topique à une surface d'une partie du corps d'un sujet qui en a besoin.

15. Formulation topique pour utilisation selon la revendication 14, dans laquelle ladite formulation topique est appliquée à une épaisseur souhaitée sur ladite partie du corps, dans laquelle lesdits agents actifs pénètrent dans la peau de ladite partie du corps et ont un effet thérapeutique synergique dans le traitement dudit état cutané, dermatologique ou épithélial, et de préférence
comprenant en outre une réapplication de ladite formulation topique toutes les heures, tous les jours, toutes les semaines, toutes les deux semaines, tous les mois ou au besoin.
